# EUROPEAN PATENT APPLICATION

(11) **EP 4 810 480 A1**
(43) Date of publication of application: **23.09.2026**
(21) Application number: 25165157.6
(22) Date of filing: 20.03.2025
(51) Int. Cl.: C07C 323/31, C07C 323/65, C07D 303/23, C07D 303/36, C07D 303/48, C07D 339/08, C07D 409/04

(54) **MONOMERS FOR THE FORMATION OF VITRIMERS**

(71) Applicant: Airbus SAS, 31700 Blagnac Cedex (FR)
(72) Inventor: Beier, Uwe, 82024 Taufkirchen (DE); Hübner, Fabian, 82024 Taufkirchen (DE); Carré, Camille, 82024 Taufkirchen (DE); Meer, Thomas, 82024 Taufkirchen (DE); Machunze, Wolfgang, 82024 Taufkirchen (DE)
(74) Representative: Isarpatent

(57) **Abstract**

The present invention relates to a monomer, a vitrimer produced using one or more of such monomers, the use of such a vitrimer, as well as a method of manufacturing a vitrimer.

## Description

### TECHNICAL FIELD OF THE INVENTION

The present invention relates to a monomer, a vitrimer produced using one or more of such monomers, the use of such a vitrimer, as well as a method of manufacturing a vitrimer.

### TECHNICAL BACKGROUND

In aviation, lightweight construction is a crucial element. Minimized structural weight is intrinsic to aviation, enabling challenging missions but also reducing the operator cost by saving fuel and finally contributing to sustainability targets. Composites are nowadays a key facilitator of optimized aircraft weight. However, there are some limitations and drawbacks of current composite technologies, namely high cost, low rate capability, insufficient composite joining technologies and last but not least the relatively high environmental footprint of a composite part itself. The environmental footprint of composites consists of the efforts to produce the raw material, the subsequent manufacturing and recycling processes. The latter can be considered to enable a credit for a second life cycle.

Composite materials for all kinds of aerospace applications are made with reinforcement fibers and a polymeric matrix. The polymeric matrix is either thermoset (TS) or thermoplastic (TP) with both advantages and disadvantages in terms of processing, thermal/mechanical properties, bonding/fusing and recycling. The two polymeric materials differ in their chemical nature.

Thermosets yield highest laminar and interlaminar quality of composites, which is required for structural applications. But the environmental footprint is high due to the extensive curing and consolidation steps, which are usually highly energy demanding, e.g. based on autoclave processes. When manufactured to final shape, bonding of thermoset is complex, time and cost demanding due to its insoluble, non-meltable and non-malleable chemical nature. Recycling approaches are non-satisfactory to date, because it requires breaking covalent chemical bonds to recover the monomers, with high energy cost.

Thermoplasts can be recycled comparably easily. However, for high laminar and interlaminar quality, high temperatures are necessary for consolidation. Additionally, the environmental footprint of high performance thermoplastic processing is amongst the highest, particularly also in view of the necessary very high temperature regimes for consolidation. In order to eliminate that energy intensive step, research on in-situ consolidation during automated fiber placement (AFP) processes has been conducted. Due to material specific limitations, particularly diffusion speed of the long chain molecules, the quality achieved is not yet on an appropriate level required for aviation.

The described processes to manufacture thermoset- and thermoplastic-based composites are not only energetically demanding, but are also time consuming. If highest qualities are required, again autoclave-manufacturing is the process of choice. Additionally, a demand for extensive hand labor for the autoclave bagging occurs.

Today's material and manufacturing processes, for both thermoplastic (TP) and thermoset (TS), get optimized using various technologies. However, detailed studies show that even in a long term perspective there are some limitations, hindering a close to zero environmental footprint.

The special nature of fiber composites are strongly benefiting from specific designs to enable lightweight construction, thereby exploiting the light-weight potential to the next level. Particularly, joining designs are benefitting from shear load designs, which are enabled by welding or adhesive bonding. Both are appropriate for thin-walled structures and a contributor to lightweight design compared to mechanical joining, but are also time / cost consuming and more importantly challenging regarding certification rules in aviation.

In the current literature the polymeric material class "vitrimers", characterized by their reversible covalent bonding networks, e.g. comprising disulfide bonds, are seen as potential enablers of easily recyclable and repairable composites. They can be seen as potential enablers for joining, bonding and thus repair and recycling to target end-of-life issues, i.e. forming the basis for easily recyclable and repairable composites. An example of vitrimers can e.g. be found in US 11713370 B1.

However, vitrimers described in literature still lack properties such as proper manufacturability, thermal stability and sustainable raw material production. Vitrimer chemistry as identified in literature and IP research are in its infancy and molecules are rather investigated because they are easy to access and not because they represent the very edge of performance characteristics anticipated and needed for their exploitation. This is true for most of the vitrimer classes and is particularly true for disulfide-based vitrimers. Most of the time they appear to be clones of currently used epoxy resins or amine hardeners. Imine bondings can achieve vitrimeric polymer properties as well, but with different temperature/mechanical performance profiles.

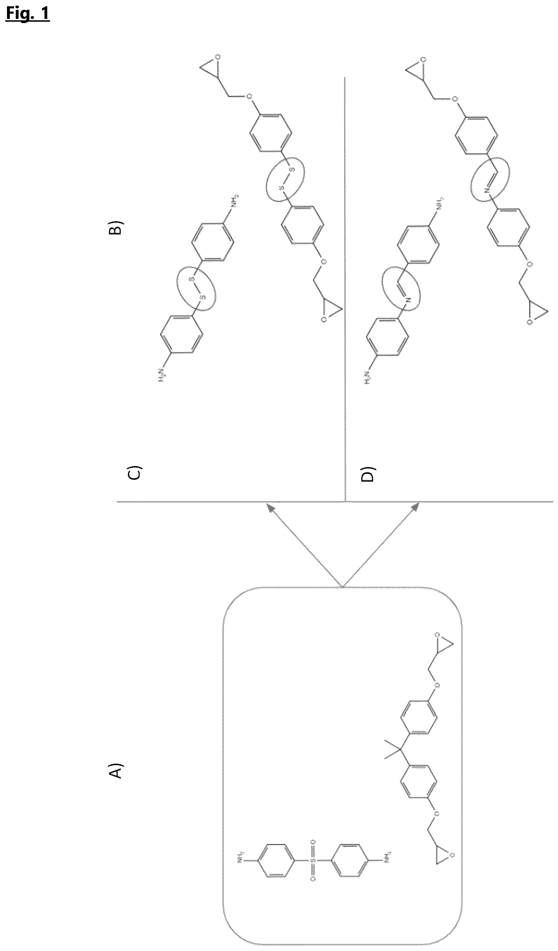

Vitrimer derivatives such as those shown in Fig. 1 work in principle, but the opportunity of improving/tailoring the glass transition temperature (Tg), vitrimer functionalities, ease of recycling, tailored curing reactivities, viscosities, enthalpies, etc. would certainly unlock a wide range of applications.

To do so, researchers currently start a more classic approach - mixing multiple vitrimers, mixing them with classic epoxy/amines and mixing additives. This certainly can lead to a more balanced characteristic, but very often improves one performance indicator by the cost of another. As some of the properties relate to the molecular nature itself, a certain limit on necessary properties cannot be exceeded.

### SUMMARY OF THE INVENTION

In order to overcome the above drawbacks, the present invention aims to improve the properties of disulfide and imine vitrimers by using engineered molecules as monomers for vitrimer formation. Thereby key properties such as service temperature and/or glass-transition temperature (Tg) and e.g. bonding speed, which are usually antagonizing, can be brought to a new level at the same time. These inventors found that vitrimers formed by the identified monomers unlock various applications while leading to the following benefits:
- reliable & certifiable bonding
- debonding on demand
- manufacturing cost reduction
- high rate production capability
- reduced environmental footprint
- improved balance between Tg and vitrimeric properties.

An aspect of the invention relates to a monomer, in particular for the formation of a polymeric network and/or vitrimer, comprising or consisting of at least one of the structures a) - c) as shown in claim 1 as appended.

Further disclosed is a method of manufacturing a polymeric network and/or vitrimer. The method comprises providing at least one monomer according to the above aspect, and polymerizing, co-polymerizing, cross-linking and/or curing the one or more monomer to obtain a polymeric network and/or vitrimer.

Also disclosed is a vitrimer or composite, produced using one or more of the monomers of the above aspect. Additionally, the invention relates to the use of such a vitrimer as adhesive, matrix resin or for surface functionalization, in particular in aircraft composites.

Further aspects and embodiments of the invention are disclosed in the dependent claims and can be taken from the following description, figures and examples, without being limited thereto.

### BRIEF SUMMARY OF THE DRAWINGS

The present invention is explained in more detail below with reference to the embodiments shown in the schematic figures:
- Fig. 1: exemplary monomers for the formation of thermosets (left) and vitrimers (right) according to the state of the art. A) classic TS, B) vitrimers, C) disulfide, D) imine.

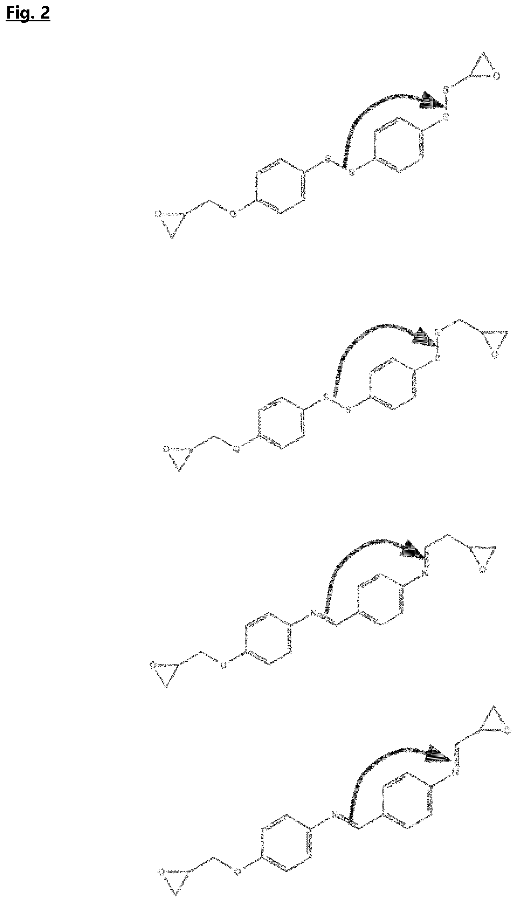

- Fig. 2: exemplary representation of compounds according to structure a). The arrows represent the displacement or additional insertion of disulfide or imine groups in the (aliphatic) part between the aromatic backbone and the end group of the monomers.

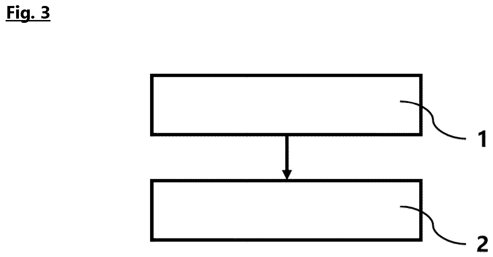

- Fig. 3: schematic representation of a method according to the invention, comprising the steps of providing at least one monomer according to the first aspect of the invention 1, and polymerizing, co-polymerizing, cross-linking and/or curing the at least one monomer to obtain the polymeric network and/or vitrimer 2.

In the figures of the drawing, elements, features and components which are identical, functionally identical and of identical action are denoted in each case by the same reference designations unless stated otherwise.

### DESCRIPTION OF EXEMPLARY EMBODIMENTS

### Definitions

Unless defined otherwise, technical and scientific terms used herein have the same meaning as commonly understood by one of ordinary skill in the art to which this invention belongs.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a", "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise.

As used herein, the terms "comprises", "comprising", "contains", "containing", "includes", "including", "has", "having" or any other variation thereof, are intended to cover a non-exclusive inclusion. For example, a process, method, article, or apparatus that comprises a list of elements is not necessarily limited to only those elements, but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

The use of the term "at least one" or "one or more" will be understood to include one as well as any quantity more than one. In addition, the use of the phrase "at least one of X, Y, and Z" will be understood to include X alone, Y alone, and Z alone, as well as any combination of X, Y, and Z.

The use of ordinal number terminology (i.e., "first", "second", "third", "fourth", etc.) is solely for the purpose of differentiating between two or more items and, unless explicitly stated otherwise, is not meant to imply any sequence or order or importance to one item over another or any order of addition.

As used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

As used herein, 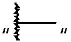 and 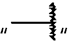 mean that the group or compound shown is linked to the neighboring group or compound of the respective structure via a covalent bond. For X₁ of structure a), for example, this means that the compounds or groups shown for X₁ are bound to L₁ or L₂ at the position labelled with the corresponding symbol.

As used herein, 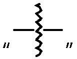 means that the respective structure is bound to a residue or a surface, in particular via a covalent bond. The residue and surface are not further limited as long as they are not detrimental to vitrimer formation from the monomers. The residue can, for example, be selected from residues of known monomers for vitrimer formation.

As used herein, 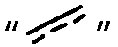 means that the bond can be a single bond 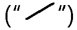 or a double bond

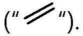

If a bond crosses or is "drawn over" another bond of a ring shown, such as 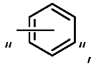 this means that the bond in question can be at any possible position of the ring.

In a first aspect, the present invention relates to a monomer comprising or consisting of at least one of structures a), b) and/or c). In some embodiments, the monomer is for the formation and/or the manufacture and/or the production of a polymeric network and/or a vitrimer. The structures a), b) and c) will be detailed in the following.

According to certain embodiments, the monomer comprises or consist of structure a): 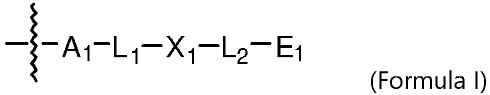 wherein
A₁ is selected from the group consisting of 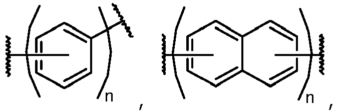 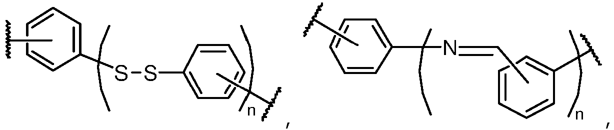 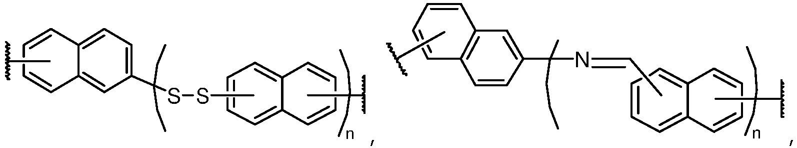 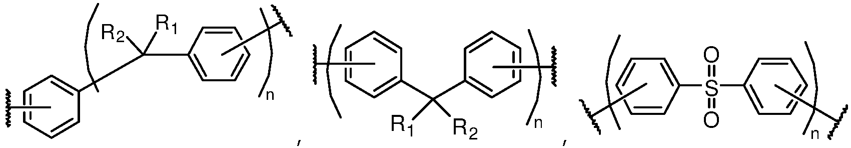 and combinations thereof, with n being an integer from 1 to 10, and R₁ and R₂ are independently selected from H, C₁ to C₆ alkyl groups and C₆ to C₂₀ aryl groups. In the groups shown for A₁, 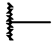 may mean that A₁ is covalently bound to 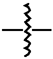 at this position and 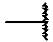 may mean that A₁ is covalently bound to L₁ at this position, or to X₁ if L₁ is a bond. According to preferred embodiments, n is 1 to 3 and more preferably 1. In preferred embodiments, R₁ and R₂ are independently selected from H and C₁ to C₃ alkyl groups, and more preferably, R₁ and R₂ are H. L₁ is selected from the group consisting of a bond, especially a covalent bond, 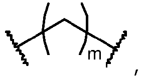 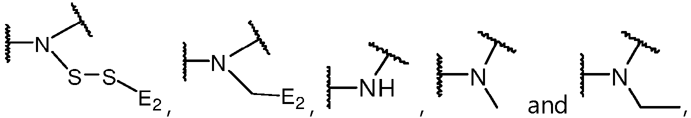 with m being an integer from 1 to 5. If L₁ is a bond, this means that A₁ is directly bound to X₁ via a covalent bond. In the groups shown for L₁, 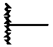 may mean that L₁ is covalently bound to A₁ at this position and 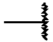 may mean that L₁ is covalently bound to X₁ at this position. According to preferred embodiments, m is 1 or 2 and more preferably 1. E₂ is 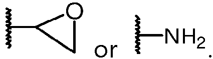 X₁ is 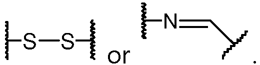 In the groups shown for X₁, 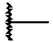 may mean that X₁ is covalently bound to L₁ at this position, or to A₁ if L₁ is a bond, and 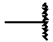 may mean that X₁ is covalently bound to L₂ at this position, or to E₁ if L₂ is a bond. L₂ is a bond, especially a covalent bond, or 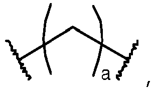 with a being an integer from 1 to 5. If L₂ is a bond, this means that X₁ is directly bound to E₁ via a covalent bond. According to preferred embodiments, a is 1 or 2 and more preferably 1. E₁ is 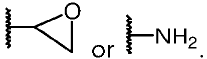 In some embodiments, the aromatic groups and/or aliphatic groups of structure a) are optionally substituted with C₁ to C₆ alkyl groups, preferably C₁ to C₃ alkyl groups, and/or C₆ to C₂₀ aryl groups, preferably C₆ to C₁₀ aryl groups.

The inventors surprisingly found that vitrimers formed from the monomers comprising or consisting of structure a) have improved vitrimeric functions as well as improved strength, stiffness and Tg. Without being bound to any particular theory, it is assumed that the positioning of the disulfide or imine group X₁ between the aromatic groups A₁, which form the backbone of the vitrimer, and the end group E₁ results in the favorable properties. In vitrimer monomers according to the state of the art, such as the compounds shown on the right side of Figure 1, the disulfide and imine groups responsible for the vitrimeric functions are positioned between the aryl groups of the aromatic backbone, which leads to a higher flexibility of the backbone and therefore to a lower Tg of the resulting vitrimer. By introducing the disulfide or imine groups in the (aliphatic) part between the aromatic backbone and the end groups of the monomer, the vitrimeric functionalities can be enhanced without reducing the thermal and mechanical properties of the vitrimer. In particular, the configuration of the monomers of the invention can improve the vitrimeric properties while maintaining the Tg. Furthermore, the monomers according to the invention enable the vitrimer material to stay high temperature resistant while having high modulus, low oxidation, low shrinkage and low water uptake by maintaining a closely cross-linked network.

According to preferred embodiments, the aromatic groups of structure a) are optionally substituted with C₁ to C₃ alkyl groups. This has a beneficial effect on the viscosity of the monomers or monomer mixtures.

According to certain embodiments, structure a) is a compound according to Formula (la):

E₃-L₄-X₂-L₃-A₁-L₁-X₁-L₂-E₁ (Formula Ia),

wherein A₁, L₁, X₁, L₂ and E₁ are as defined above. It is understood that 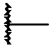 in this case for the groups shown for A₁ may mean that A₁ is covalently bound to L₃ at this position, or to X₂ if L₃ is a bond. The above statements apply analogously here. L₃ is selected from the group consisting of a bond, especially a covalent bond, 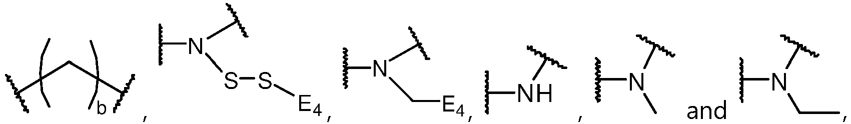 with b being an integer from 1 to 5. If L₃ is a bond, this means that A₁ is directly bound to X₂ via a covalent bond. In the groups shown for L₃, 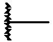 may mean that L₃ is covalently bound to A₁ at this position and 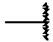 may mean that L₃ is covalently bound to X₂ at this position. According to preferred embodiments, b is 1 or 2 and more preferably 1. E₄ is 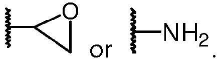 X₂ is 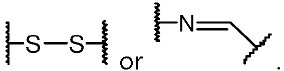 In the groups shown for X₂, 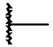 may mean that X₂ is covalently bound to L₃ at this position, or to A₁ if L₃ is a bond, and 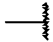 may mean that X₂ is covalently bound to L₄ at this position, or to E₃ if L₄ is a bond. L₄ is a bond, especially a covalent bond, or 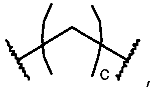 with c being an integer from 1 to 5. If L₄ is a bond, this means that E₃ is directly bound to X₂ via a covalent bond. According to preferred embodiments, c is 1 or 2 and more preferably 1. E₃ is selected from the group consisting of 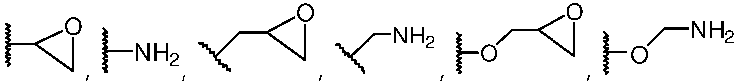 and H. In these embodiments, the above-mentioned advantages and effects of the invention are particularly pronounced.

Alternatively or in addition, structure a) may be a compound according to Formula (Ib):

E₃-A₁-L₁-X₁-L₂-E₁ (Formula Ib)

wherein A₁, L₁, X₁, L₂, E₁ and E₃ are as defined above. It is understood that 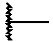 in this case for the groups shown for A₁ may mean that A₁ is covalently bound to E₃ at this position. The above statements apply analogously here. In these embodiments, the above-mentioned advantages and effects of the invention are particularly pronounced.

According to certain embodiments, A₁ is selected from the group consisting of 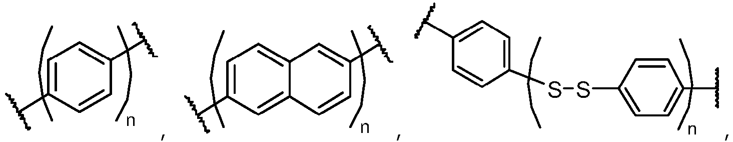 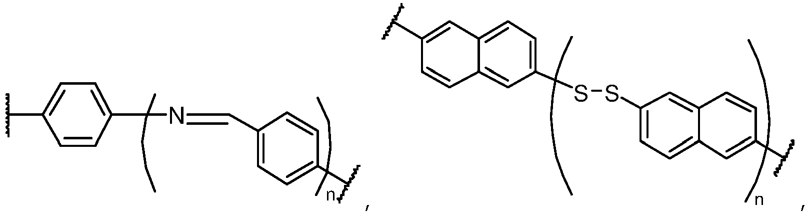 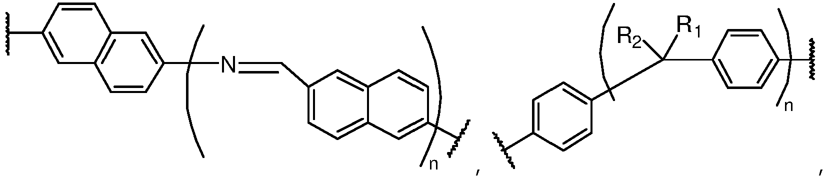 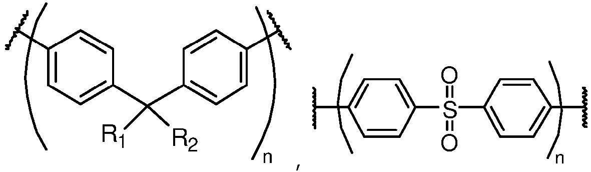 and combinations thereof.

According to preferred embodiments, A₁ is selected from the group consisting of 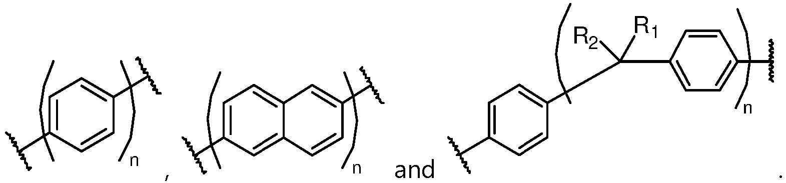 According to further preferred embodiments, A₁ is selected from the group consisting of

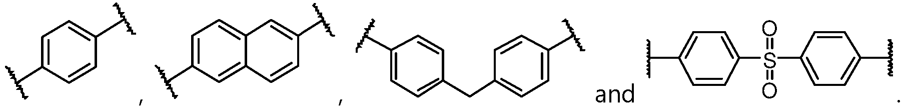

The above statements apply analogously here. In these embodiments, the above-mentioned advantages and effects of the invention are particularly pronounced. In particular, the Tg of the vitrimer can be further increased if A₁ does not contain any disulfide or imine groups. In some embodiments, when E₁, E₂, E₃ and/or E₄ is 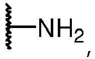 the remaining groups are selected such that the amine group 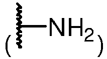 is not directly bound to a sulfur, oxygen and/or nitrogen atom. In particular, the remaining groups may be selected such that the amine group 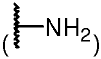 is not directly bound to an oxygen or nitrogen atom. According to certain embodiments, structure a) contains either amine or epoxy groups. In other words, in some embodiments, structure a) does not have both amine and epoxy groups. In some embodiments, E₁ is 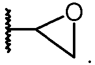 Alternatively or in addition, E₂ may be 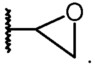 Alternatively or in addition, E₄ may be 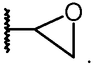 In preferred embodiments, E₁, E₂ and E₄ are 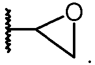 In certain embodiments, E₃ is selected from the group consisting of

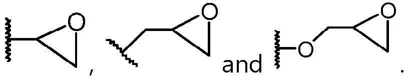

According to preferred embodiments, E₃ is 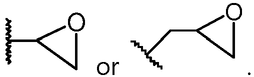 In these embodiments, the above-mentioned advantages and effects of the invention are particularly pronounced.

In some embodiments, X₁ is 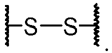 Alternatively or in addition, X₂ may be 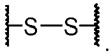 According to preferred embodiments, X₁ and X₂ are 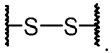 In these embodiments, the above-mentioned advantages and effects of the invention are particularly pronounced. According to certain embodiments, structure a) is selected from the group consisting of 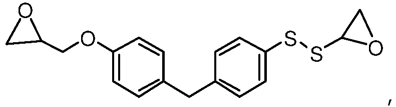 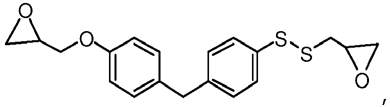 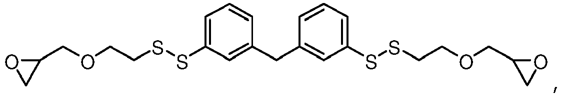 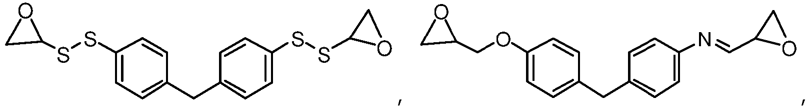 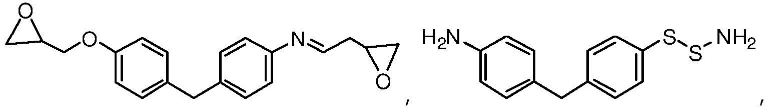 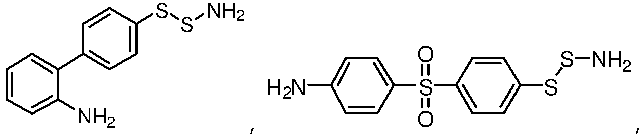 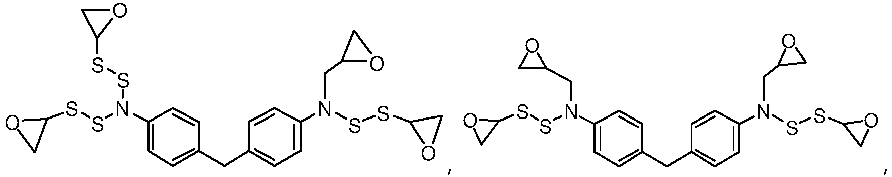 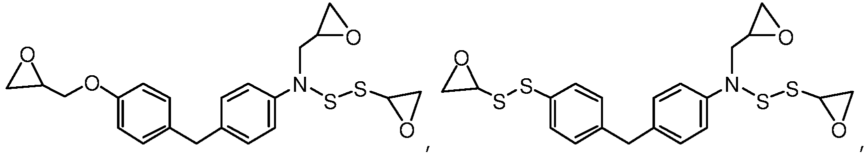 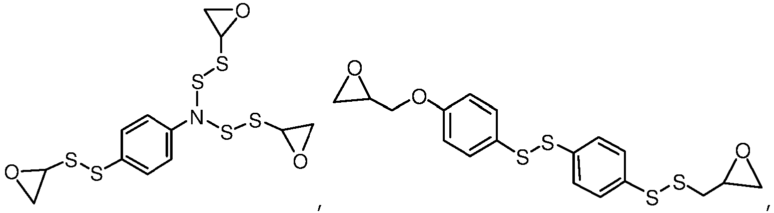 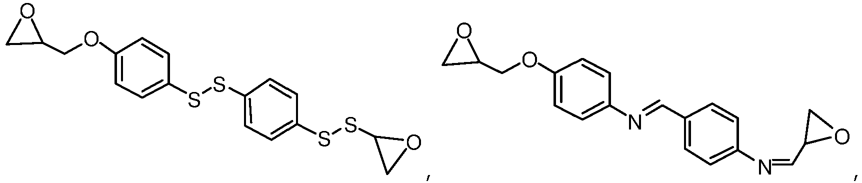 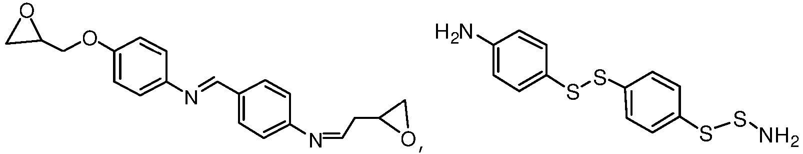 and 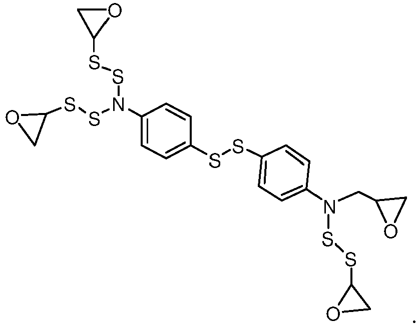 In some embodiments, structure a) is selected from the group consisting of 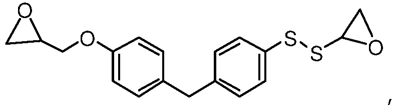 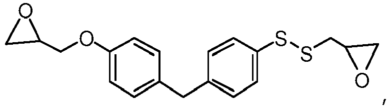 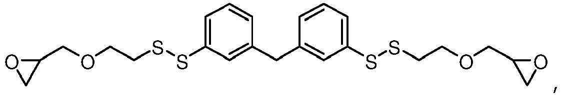 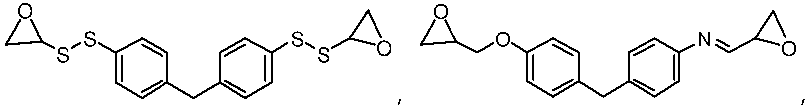 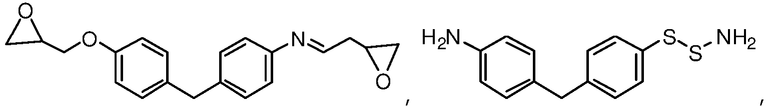 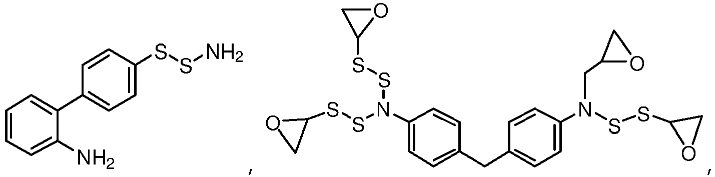 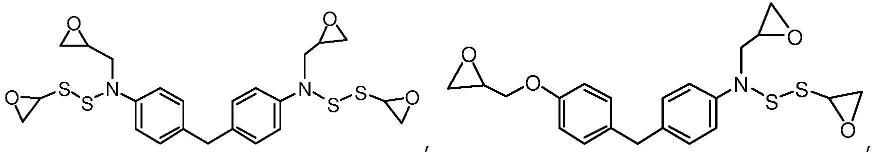 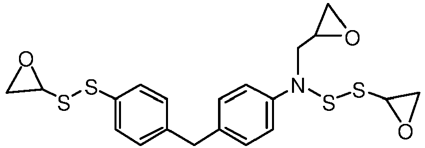 and 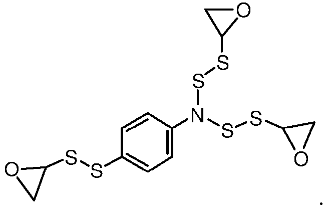 According to certain embodiments, structure a) is selected from the group consisting of 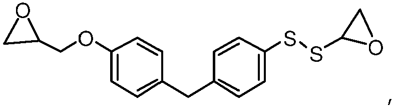 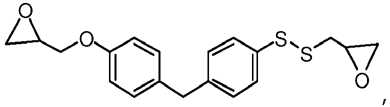 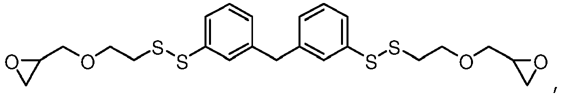 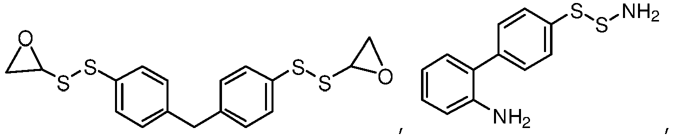 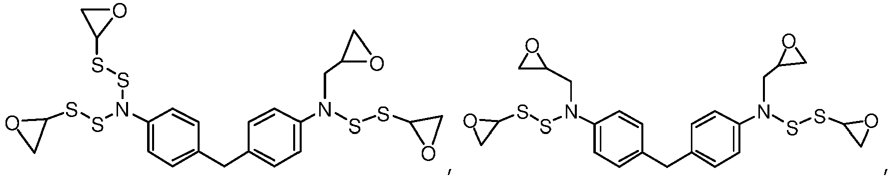 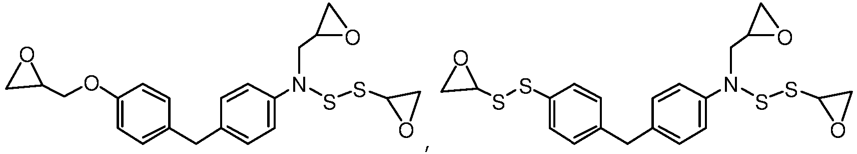 and 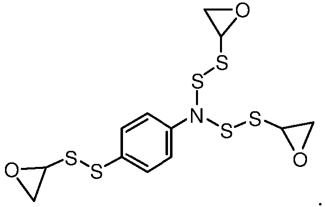 In some embodiments, structure a) is selected from the group consisting of 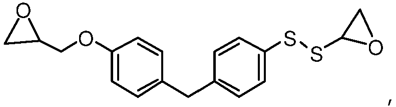 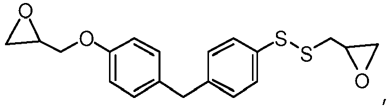 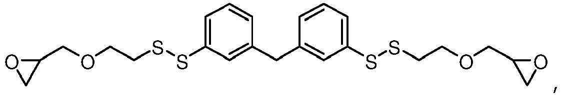 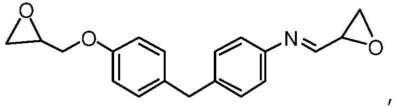 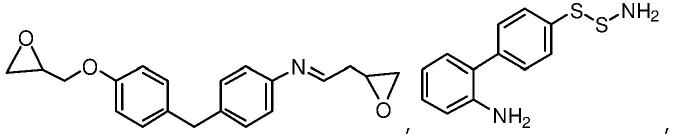 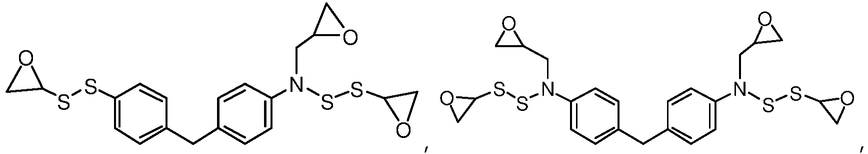 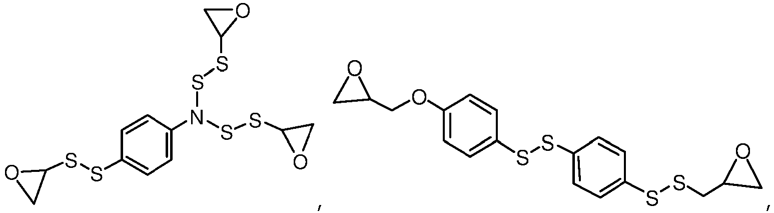 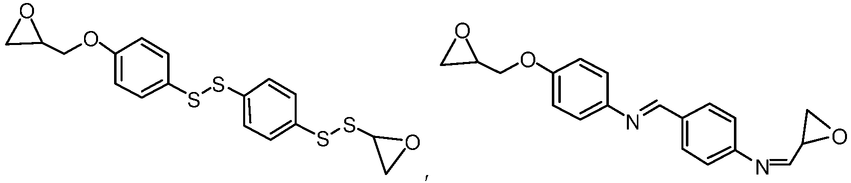 and 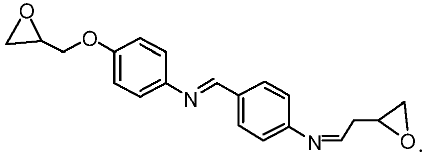 According to certain embodiments, structure a) is selected from the group consisting of 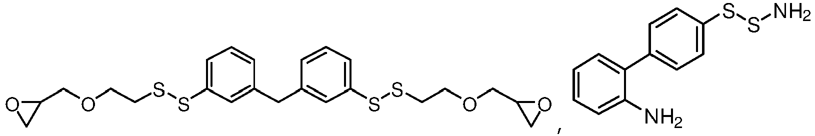 and 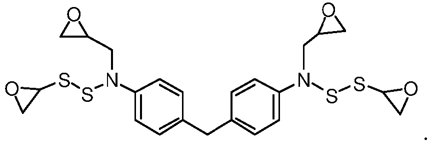 In these embodiments, the above-mentioned advantages and effects of the invention are particularly pronounced.

According to some embodiments, the monomer comprises or consist of structure b): 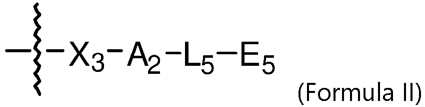 wherein A₂ is selected from the group consisting of 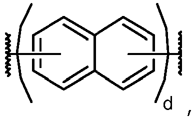 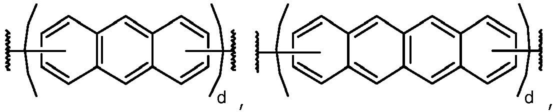 and combinations thereof, with d being an integer from 1 to 10. In the groups shown for A₂, 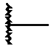 may mean that A₂ is covalently bound to X₃ at this position and 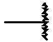 may mean that A₂ is covalently bound to L₅ at this position, or to E₅ if L₅ is a bond. According to preferred embodiments, d is 1 to 3 and more preferably 1. X₃ is 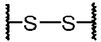 or 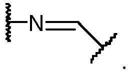 In the groups shown for X₃, 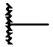 may mean that X₃ is covalently bound to 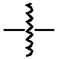 at this position, and 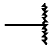 may mean that X₃ is covalently bound to A₂ at this position. L₅ is selected from the group consisting of a bond, especially a covalent bond, 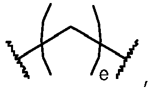 with e being an integer from 1 to 5, 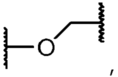 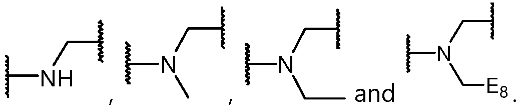 Es is 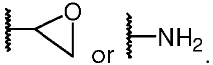 If L₅ is a bond, this means that A₂ is directly bound to E₅ via a covalent bond. In the groups shown for L₅, 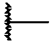 may mean that L₅ is covalently bound to A₂ at this position and 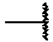 may mean that L₅ is covalently bound to E₅ at this position. According to preferred embodiments, e is 1 or 2 and more preferably 1. E₅ is

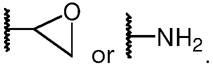

In some embodiments, the aromatic groups and/or aliphatic groups of structure b) are optionally substituted with C₁ to C₆ alkyl groups, preferably C₁ to C₃ alkyl groups, and/or C₆ to C₂₀ aryl groups, preferably C₆ to C₁₀ aryl groups.

The inventors surprisingly found that vitrimers formed from the monomers comprising or consisting of structure b) have improved vitrimeric functions as well as improved strength, stiffness and Tg. Without being bound to any particular theory, it is assumed that the introduction of multiple rings such as double or triple rings in the monomer can compensate for the negative effects of the flexibility caused by the disulfide or imine groups in the aromatic part of the vitrimer on the Tg or even lead to an increase in the Tg. By increasing the rigidity through the multiple rings, more vitrimeric functionalities can be made available without affecting the Tg. In particular, the configuration of the monomers of the invention can improve the vitrimeric properties while maintaining or increasing the Tg. Furthermore, the monomers according to the invention enable the vitrimer material to stay high temperature resistant while having high modulus, low oxidation, low shrinkage and low water uptake by maintaining a closely cross-linked network.

According to preferred embodiments, the aromatic groups of structure b) are optionally substituted with C₁ to C₃ alkyl groups. This has a beneficial effect on the viscosity of the monomers or monomer mixtures.

In certain embodiments, structure b) is a compound according to Formula (Ila):

E₆-L₆-A₃-X₃-A₂-L₅-E₅ (Formula (IIa)

wherein X₃, A₂, L₅ and E₅ are as defined above. It is understood that 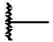 in this case for the groups shown for X₃ may mean that X₃ is covalently bound to A₃ at this position. The above statements apply analogously here. A₃ is selected from the group consisting of 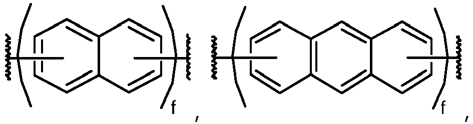 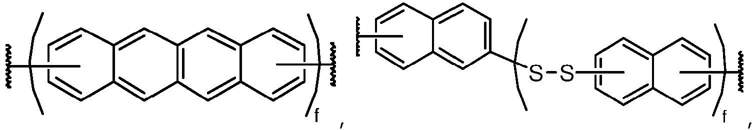 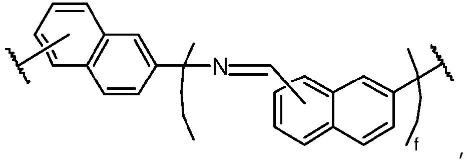 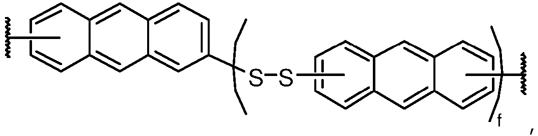 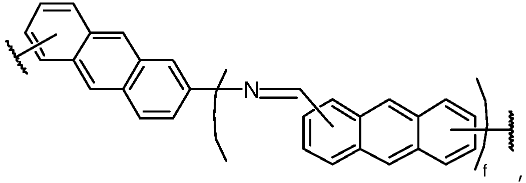 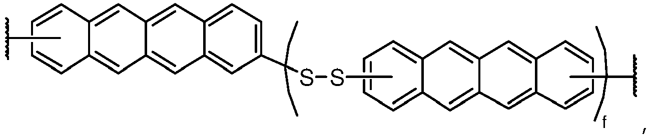 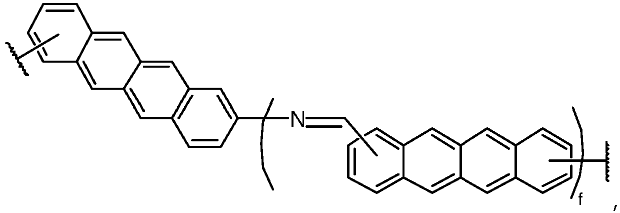 and combinations thereof, with f being an integer from 1 to 10. In the groups shown for A₃, 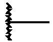 may mean that A₃ is covalently bound to L₆ at this position, or to E₆ if L₆ is a bond, and 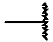 may mean that A₃ is covalently bound to X₃ at this position. According to preferred embodiments, f is 1 to 3 and more preferably 1. L₆ is selected from the group consisting of a bond, especially a covalent bond, 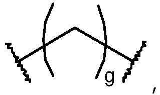 with g being an integer from 1 to 5, 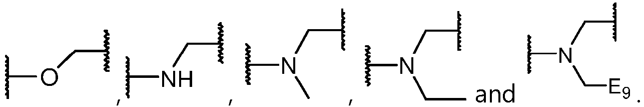 E₉ is 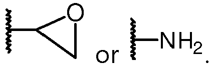 If L₆ is a bond, this means that A₃ is directly bound to E₆ via a covalent bond. In the groups shown for L₆, 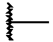 may mean that L₆ is covalently bound to A₃ at this position and 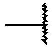 may mean that L₆ is covalently bound to E₆ at this position. According to preferred embodiments, g is 1 or 2 and more preferably 1. E₆ is 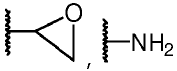 or H. In these embodiments, the above-mentioned advantages and effects of the invention are particularly pronounced.

In some embodiments, A₂ is selected from the group consisting of 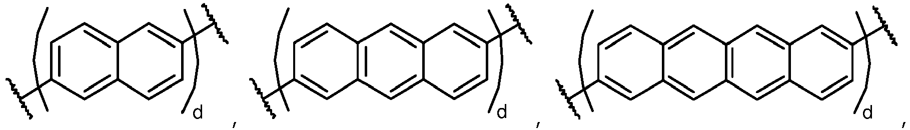 and combinations thereof. According to preferred embodiments, A₂ is selected from the group consisting of

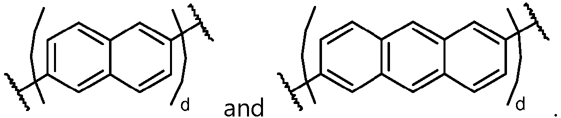

According to further preferred embodiments, A₂ is 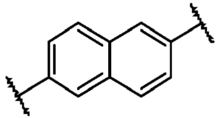 or 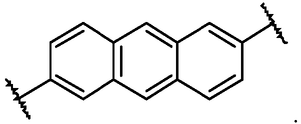 The above statements apply analogously here. In these embodiments, the above-mentioned advantages and effects of the invention are particularly pronounced.

According to certain embodiments, A₃ is selected from the group consisting of 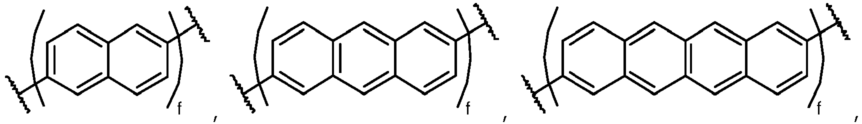 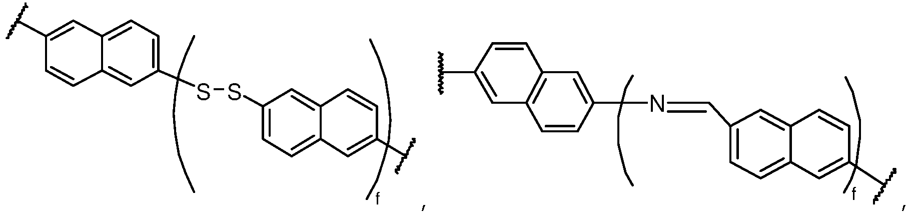 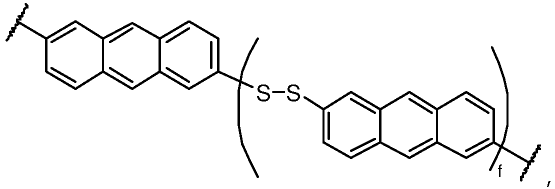 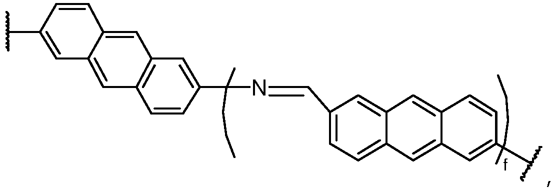 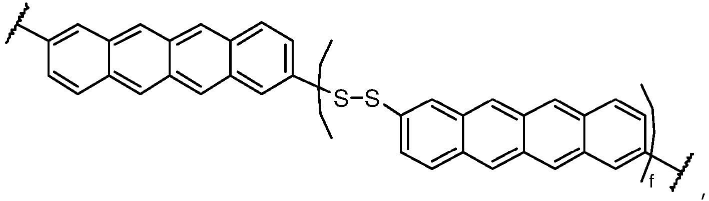 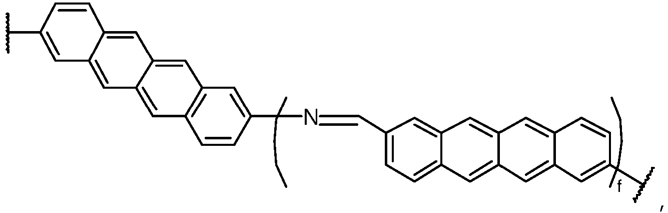 and combinations thereof. In preferred embodiments, A₃ is selected from the group consisting of 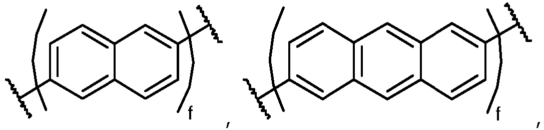 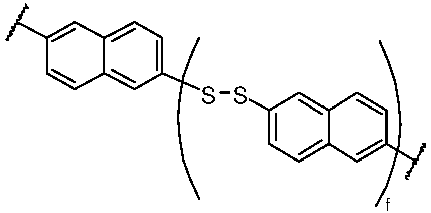 and 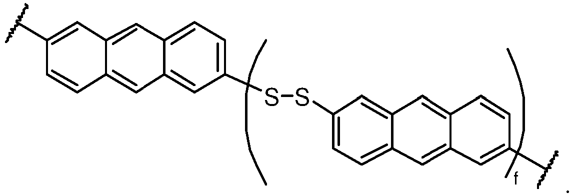 According to further preferred embodiments, A₃ is selected from the group consisting of 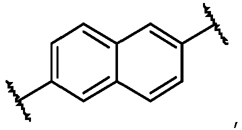 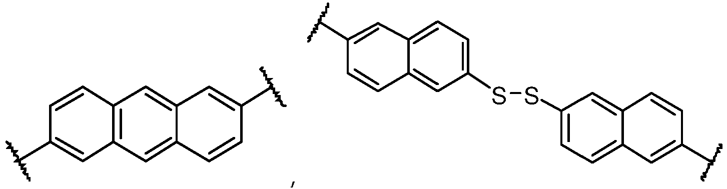 and 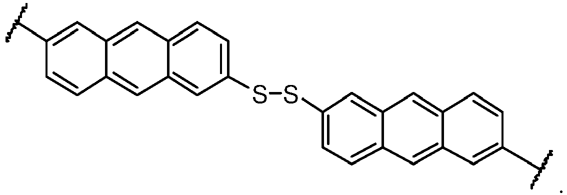 The above statements apply analogously here. In these embodiments, the above-mentioned advantages and effects of the invention are particularly pronounced.

In some embodiments, when E₅ and/or E₆ is 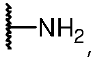 the remaining groups are selected such that the amine group 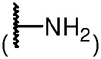 is not directly bound to a sulfur, oxygen and/or nitrogen atom. In particular, the remaining groups may be selected such that the amine group 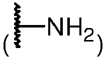 is not directly bound to an oxygen or nitrogen atom. According to certain embodiments, structure b) contains either amine or epoxy groups. In other words, in some embodiments, structure b) does not have both amine and epoxy groups. In some embodiments, E₅ is 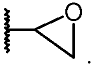 Alternatively or in addition, E₆ may be 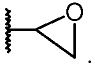 Alternatively or in addition, E₈ may be 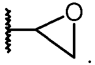 Alternatively or in addition, E₉ may be 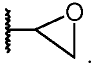 In preferred embodiments, E₅, E₆, E₈ and E₉ are 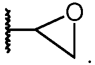 In some embodiments, X₃ is 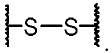 In these embodiments, the above-mentioned advantages and effects of the invention are particularly pronounced.

In some embodiments, structure b) is selected from the group consisting of 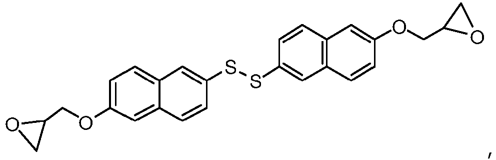 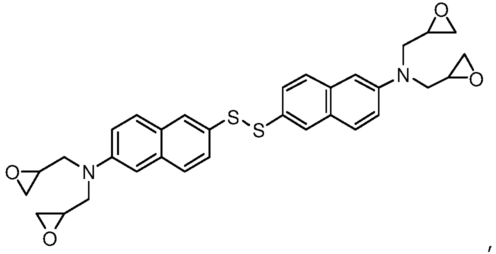 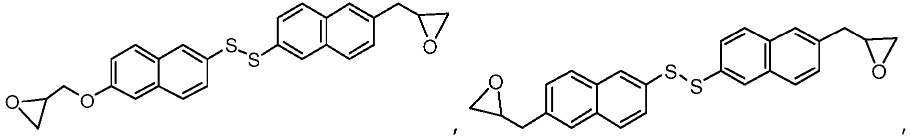 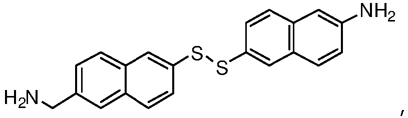 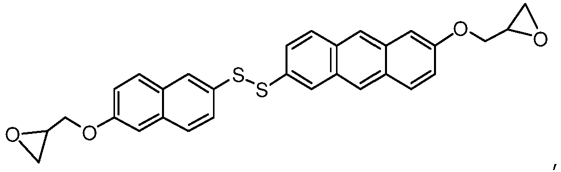 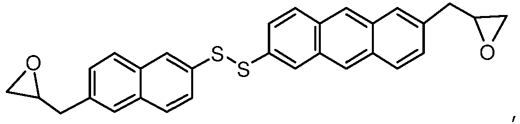 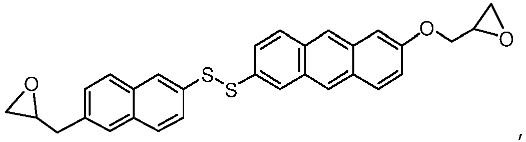 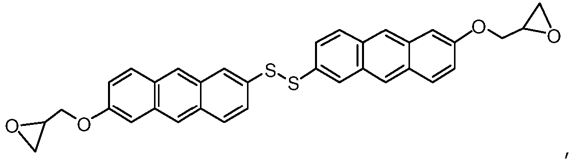 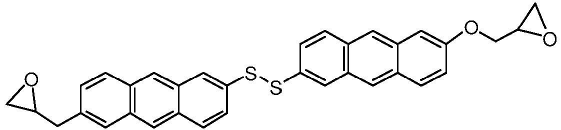 and

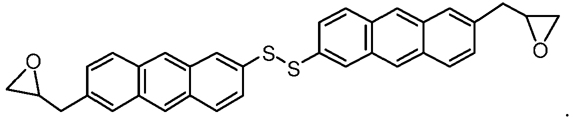

According to some embodiments, structure b) is selected from the group consisting of 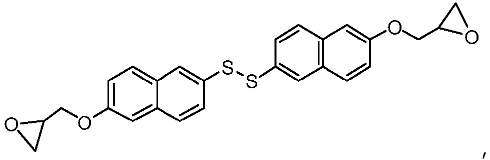 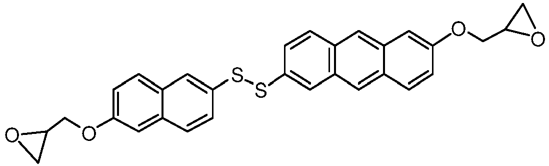 and 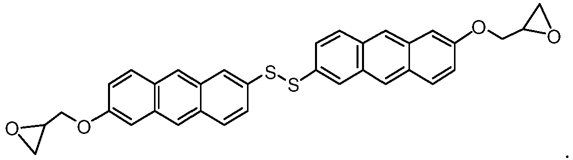 In certain embodiments, structure b) is 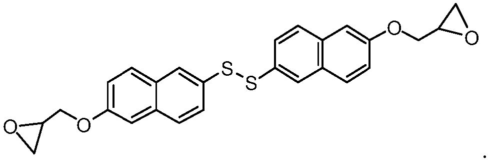 In these embodiments, the above-mentioned advantages and effects of the invention are particularly pronounced.

According to some embodiments, the monomer comprises or consist of structure c):

G₂ -Z-G₁ (Formula III)

wherein Z is selected from the groups consisting of 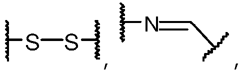 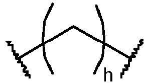 with h being an integer from 1 to 5, 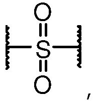 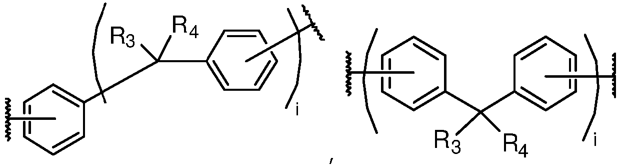 and 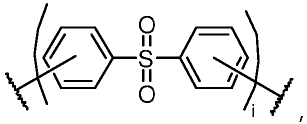 with i being an integer from 1 to 5, and R₃ and R₄ are independently selected from H, C₁ to C₆ alkyl groups and C₆ to C₂₀ aryl groups. In the groups shown for Z, 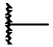 may mean that Z is covalently bound to G₁ at this position and 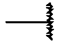 may mean that Z is covalently bound to G₂ at this position.

According to preferred embodiments, h is 1 to 3 and more preferably 1. In preferred embodiments, i is 1 to 3 and more preferably 1. According to preferred embodiments, R₃ and R₄ are independently selected from H and C₁ to C₃ alkyl groups, and more preferably, R₃ and R₄ are H. G₁ and G₂ are each independently selected from the group consisting of 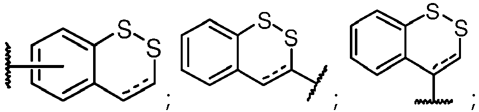 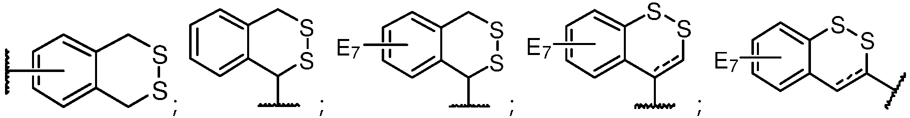 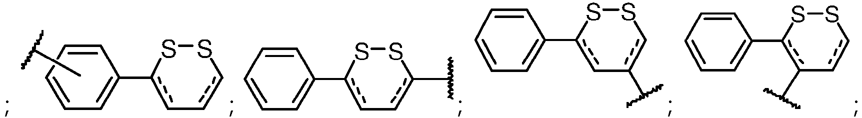 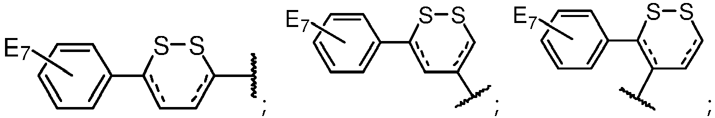 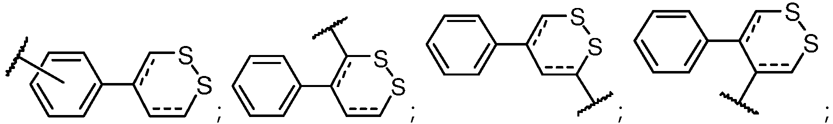 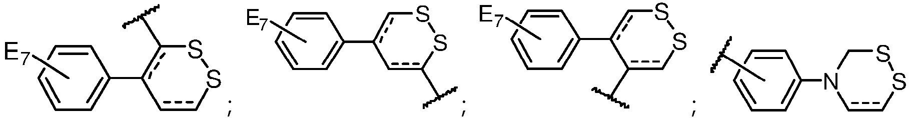 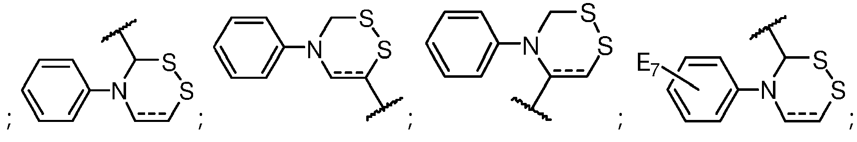 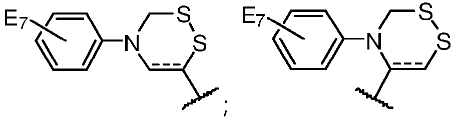 and analogue structures with four-, five-, seven- and/or eight-membered rings, preferably five- and/or seven-membered rings and more preferably five-membered rings. In this context, "analogue structures" refer to structures as shown for G1 and G2, but with at least one of the six-membered rings replaced by a four, five-, seven- and/or eight-membered ring. An example for such an analogue structure is 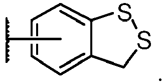 E₇ is selected from the group consisting of 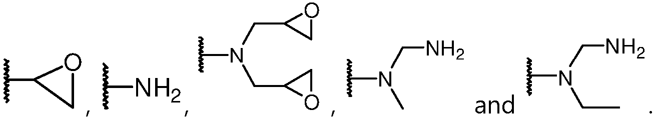 In some embodiments, the aromatic groups and/or aliphatic groups of structure c) are optionally substituted with C₁ to C₆ alkyl groups, preferably C₁ to C₃ alkyl groups, and/or C₆ to C₂₀ aryl groups, preferably C₆ to C₁₀ aryl groups.

The inventors surprisingly found that vitrimers formed from the monomers comprising or consisting of structure c) have improved vitrimeric functions as well as improved strength, stiffness and Tg. Without being bound to any particular theory, it is assumed that the positioning of the disulfide group in cyclic and rigid or stiff structures of the monomer results in the favorable properties. In particular, the groups G₁ and G₂ shown above lead to a higher stiffness and thus a higher Tg as well as a higher thermal resistance of the vitrimer. Furthermore, as explained above, in vitrimer monomers according to the state of the art, the disulfide groups responsible for the vitrimeric functions are positioned between the aryl groups of the aromatic backbone, which leads to a higher flexibility of the backbone and therefore to a lower Tg of the resulting vitrimer. By introducing the disulfide groups in cyclic and rigid parts of the monomer, the vitrimeric functionalities can be enhanced without reducing the thermal and mechanical properties of the vitrimer. In particular, the configuration of the monomers of the invention can improve the vitrimeric properties while maintaining or increasing the Tg. Furthermore, the monomers according to the invention enable the vitrimer material to stay high temperature resistant while having high modulus, low oxidation, low shrinkage and low water uptake by maintaining a closely crosslinked network. A further advantage of the monomers comprising or consisting of structure c) is that these monomers can be formed into a polymeric network and/or vitrimer by means of homopolymerization of the disulfide groups.

According to preferred embodiments, the aromatic groups of structure c) are optionally substituted with C₁ to C₃ alkyl groups. This has a beneficial effect on the viscosity of the monomers or monomer mixtures. In certain embodiments, Z is selected from the groups consisting of 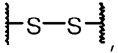 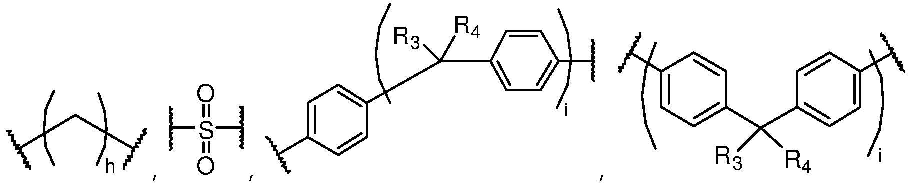 and 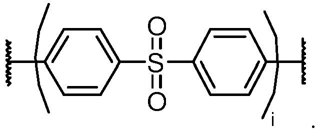 According to preferred embodiments, Z is selected from the group consisting of 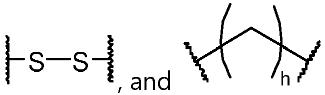 with h being 1 or 2. The above statements apply analogously here. In these embodiments, the above-mentioned advantages and effects of the invention are particularly pronounced.

In some embodiments, G₁ and G₂ are each independently selected from the group consisting of 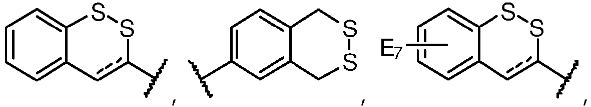 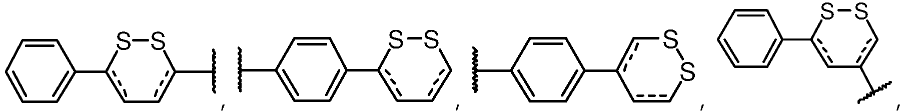 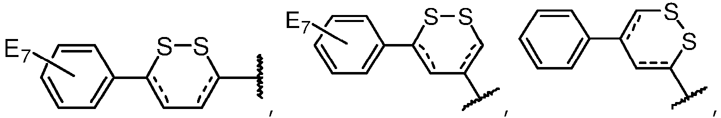 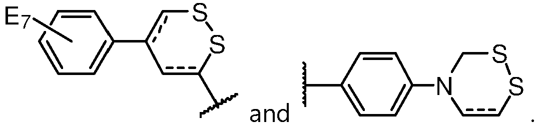 According to preferred embodiments, G₁ and G₂ are each independently selected from the group consisting of 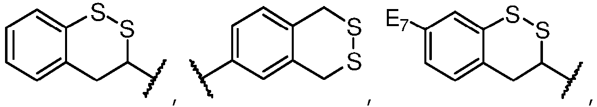 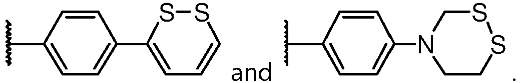 In preferred embodiments, E₇ is 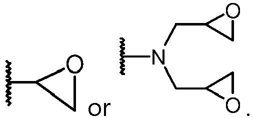 The above statements apply analogously here. In these embodiments, the above-mentioned advantages and effects of the invention are particularly pronounced.

According to certain embodiments, structure c) contains either amine or epoxy groups. In other words, in some embodiments, structure c) does not have both amine and epoxy groups.

In certain embodiments, structure c) is selected from the group consisting of 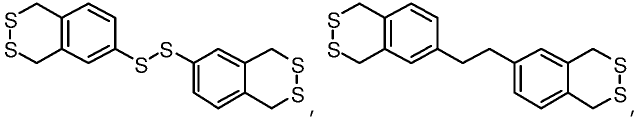 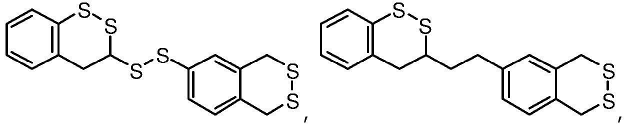 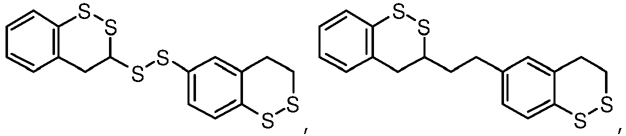 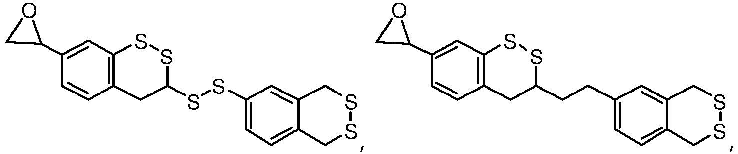 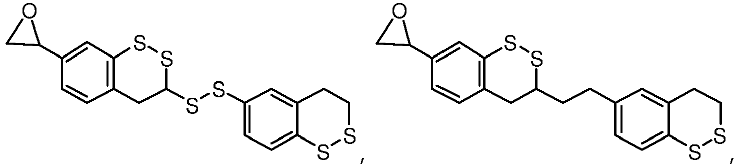 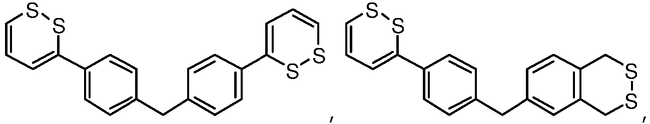 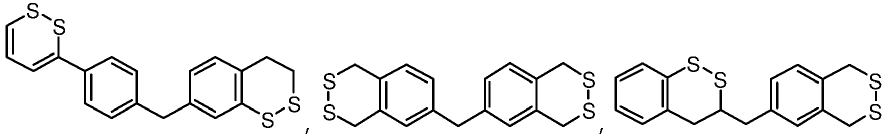 and 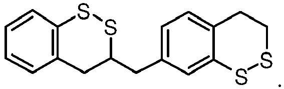 According to some embodiments, structure c) is selected from the group consisting of 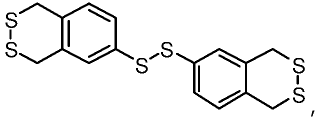 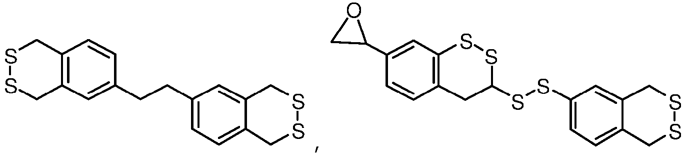 and 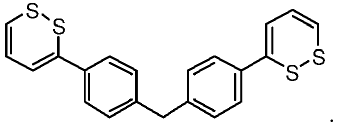 In some embodiments, structure c) is 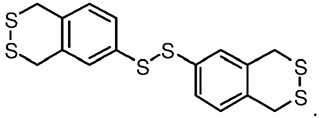 In these embodiments, the above-mentioned advantages and effects of the invention are particularly pronounced.

According to certain embodiments, the monomer according to the invention comprises or consists of a compound or a mixture of compounds, especially a mixture of two or more compounds, selected from the group consisting of compounds according to structure a), compounds according to structure b) and compounds according to structure c). In some embodiments, the monomer according to the invention comprises or consists of a compound or a mixture of compounds, especially a mixture of two or more compounds, selected from the group consisting of compounds according to structure a) and compounds according to structure c). In some embodiments, the monomer according to the invention comprises or consists of a compound or a mixture of compounds, especially a mixture of two or more compounds, selected from the group consisting of compounds according to structure a) and compounds according to structure b). According to some embodiments, the monomer according to the invention comprises or consists of a compound or a mixture of compounds, especially a mixture of two or more compounds, selected from the group consisting of compounds according to structure b) and compounds according to structure c).

In certain embodiments, the monomer according to the invention is a compound according to structure a). According to some embodiments, the monomer according to the invention is a compound according to structure b). According to certain embodiments, the monomer according to the invention is a compound according to structure c).

According to some embodiments, the monomer according to the invention comprises or consists of a mixture of compounds, wherein the mixture comprises or consists of at least one compound according to structure a), at least one compound according to structure b) and at least one compound according to structure c). In certain embodiments, the monomer according to the invention comprises or consists of a mixture of compounds, wherein the mixture comprises or consists of at least one compound according to structure a) and at least one compound according to structure b). According to certain embodiments, the monomer according to the invention comprises or consists of a mixture of compounds, wherein the mixture comprises or consists of at least one compound according to structure a) and at least one compound according to structure c). In some embodiments, the monomer according to the invention comprises or consists of a mixture of compounds, wherein the mixture comprises or consists of at least one compound according to structure b) and at least one compound according to structure c).

The monomers of the present invention can be polymerized and/or cross-linked in order to form and/or produce a polymeric network and/or a vitrimer and/or a composite material. In some embodiments, the monomers comprise epoxy groups. The monomers may be polymerized by the epoxy groups present in the monomers, for example by cationic polymerization. Alternatively or in addition, the monomers comprising epoxy groups may be cross-linked, for example by cross-linking agents and/or hardeners comprising amine, hydroxyl and/or thiol groups. According to certain embodiments, the monomers comprise a first monomer comprising at least one amine group and a second monomer comprising at least one epoxy group. The monomers may be polymerized by the epoxy and amine groups present in the monomers, for example by polyaddition and/or step-growth polymerization. Alternatively or in addition, the monomers comprising epoxy and/or amine groups may be cross-linked, for example by cross-linking agents and/or hardeners comprising amine, epoxy, hydroxyl and/or thiol groups. According to some embodiments, the monomers comprise disulfide groups. The monomers may be cross-linked, for example by cross-linking agents and/or hardeners comprising thiol groups, or by disulfide-disulfide exchange reactions. In some embodiments, the monomers comprise cyclic disulfide groups. As used herein, "cyclic disulfide groups" refer to heterocyclic groups containing a disulfide group as part of the cycle or ring. In particular, the groups for G₁ and G₂ contain such cyclic disulfide groups. The monomers may be polymerized by the cyclic disulfide groups present in the monomers, for example by ring-opening polymerization. Alternatively or in addition, the monomers may be cross-linked, for example by cross-linking agents and/or hardeners comprising thiol groups, or by disulfide-disulfide exchange reactions.

In a second aspect, the present invention relates to a composition comprising one or more monomers according to the first aspect of the present invention. Reference is made to the above statements on the first aspect of the invention, which apply analogously here. According to certain embodiments, the composition is an uncured mixture and/or a prepreg. The composition can be applied to a substrate and then cured. Curing can be carried out, for example, by temperature treatment and/or the addition of a crosslinking agent and/or an initiator and/or a catalyst.

In certain embodiments, the composition further comprises a cross-linking agent and/or a hardener. The cross-linking agent and/or hardener may be selected from the group consisting of 1,3,5-benzene trithiol (BTT), 1,4-benzene dithiol (BDT), 4,4'-biphenyl dithiol (BPDT), 2-thioaminophenol, 3-thioaminophenol, 4-thioaminophenol, 2,2'-dithiodianiline, 4,4'-dithiodianiline and 2-Amino-4-mercapto-6-methyl-1,3,5-triazine. In some embodiments, the composition comprises or consists of two or more monomers according to the first aspect of the present invention and the cross-linking agent and/or hardener. In these embodiments, the above-mentioned advantages and effects of the invention are particularly pronounced.

In a third aspect, the present invention relates to a method of manufacturing a polymeric network and/or a vitrimer. The method comprises the steps of providing at least one monomer according to the first aspect of the invention 1 and polymerizing, co-polymerizing, cross-linking and/or curing the monomer to obtain an oligomer and/or a polymer and/or the polymeric network and/or the vitrimer 2. Alternatively or in addition, the method may comprise the steps of providing the composition according to the second aspect of the invention 1 and polymerizing, co-polymerizing, cross-linking and/or curing the composition to obtain an oligomer and/or a polymer and/or the polymeric network and/or the vitrimer 2. Reference is made to the above statements on the first and second aspects of the invention, which apply analogously here. According to some embodiments, the method is a method of manufacturing a vitrimer and comprises the steps of providing at least one monomer according to the first aspect of the invention 1 and polymerizing and/or cross-linking the at least one monomer to obtain the vitrimer 2. In certain embodiments, the method is a method of manufacturing a vitrimer and comprises the steps of providing the composition according to the second aspect of the invention 1 and polymerizing and/or cross-linking the composition to obtain the vitrimer 2.

The polymerization of the monomer and/or composition is not particularly limited. According to certain embodiments, the polymerization is selected from the group consisting of cationic polymerization, polyaddition, step-growth polymerization and ring-opening polymerization, preferably from the group consisting of polyaddition, step-growth polymerization and ring-opening polymerization.

In some embodiments, the at least one monomer comprises epoxy groups and the monomers are polymerized via cationic polymerization.

Alternatively or in addition, a first monomer according the first aspect of the invention and a second monomer according to the first aspect of the invention are provided, wherein the first monomer comprises at least one amine group and the second monomer comprises at least one epoxy group. The monomers may be polymerized via step-growth polymerization.

Alternatively or in addition, the at least one monomer may comprise cyclic disulfide groups and/or may comprise or consist of at least one compound according to structure c) and the monomers may be polymerized via ring-opening polymerization.

Alternatively or in addition, the polymerization and/or the cross-linking and/or curing may be conducted by the addition of a cross-linking agent and/or a hardener. In this context, reference is made to the above statements on the first and second aspects of the invention, which apply analogously here. The cross-linking agent and/or hardener may be selected from the groups consisting of 1,3,5-benzene trithiol (BTT), 1,4-benzene dithiol (BDT), 4,4'-biphenyl dithiol (BPDT), 2-thioaminophenol, 3-thioaminophenol, 4-thioaminophenol, 2,2'-dithiodianiline, 4,4'-dithiodianiline and 2-Amino-4-mercapto-6-methyl-1,3,5-triazine.

In a fourth aspect, the present invention relates to a polymeric network or vitrimer or composite, produced using one or more of the monomers according to the first aspect of the invention and/or the composition according to the second aspect of the invention. In some embodiments, the polymeric network or vitrimer is produced by the method according to the third aspect of the invention. According to certain embodiments, the polymeric network or vitrimer is produced by polymerizing and/or cross-linking one or more of the monomers according to the first aspect of the invention. In some embodiments, the polymeric network or vitrimer is produced by curing and/or polymerizing and/or cross-linking the composition according to the second aspect of the invention. Reference is made to the above statements on the first, second and third aspects of the invention, which apply analogously here.

In a fifth aspect, the present invention relates to a polymeric network or vitrimer or composite, obtainable and/or obtained from one or more of the monomers according to the first aspect of the invention and/or from the composition according to the second aspect of the invention. In some embodiments, the polymeric network or vitrimer is produced by the method according to the third aspect of the invention. According to certain embodiments, the polymeric network or vitrimer is obtainable and/or obtained through polymerizing and/or cross-linking one or more of the monomers according to the first aspect of the invention. In some embodiments, the polymeric network or vitrimer is obtainable and/or obtained through curing and/or polymerizing and/or cross-linking the composition according to the second aspect of the invention. Reference is made to the above statements on the first, second, third and fourth aspects of the invention, which apply analogously here.

In a sixth aspect, the present invention relates to a composite, comprising a substrate and at least one vitrimer covalently bound to the substrate. According to certain embodiments, the at least one vitrimer comprises or consists of at least one monomer according to the first aspect of the invention. In some embodiments, the at least one vitrimer is a vitrimer according to the fourth or fifth aspect of the invention. In some embodiments, the substrate comprises or consists of a material selected from the group consisting of a thermoplastic, a thermoset, a metal and a ceramic. The substrate may be in the form of a core or a layer. In some embodiments, the material is activated before forming the covalent bond, e.g. by thermal activation, irradiation, etc. This may especially be the case if the material is a metal or ceramic. The activation may be conducted to provide functional groups for forming the covalent bond. According to certain embodiments, the substrate comprises or consists of a thermoplastic or a thermoset, or mixtures thereof, preferably a thermoset. In some embodiments, the covalent bond between the vitrimer and the substrate is formed by reaction of epoxy, amine, disulfide and/or cyclic disulfide groups of the vitrimer and functional groups of the substrate or material of the substrate. The functional groups may be selected from the group consisting of hydroxyl, epoxy, amine and thiol groups. Reference is made to the above statements on the first, second, third, fourth and fifth aspects of the invention, which apply analogously here.

In a sixth aspect, the present invention relates to the use of a vitrimer as adhesive, matrix resin and/or for surface functionalization and/or the use of a composition according to the second aspect of the invention as adhesive, matrix resin and/or for surface functionalization. According to certain embodiments, the vitrimer comprises or consists of at least one monomer according to the first aspect of the invention. In some embodiments, the vitrimer is a vitrimer according to the fourth or fifth aspect of the invention. In preferred embodiments, the vitrimer is used as adhesive, matrix resin and/or for surface functionalization in composites and especially in aircraft composites. The favorable balance between Tg and vitrimeric properties of the vitrimers of the present invention, as described above, makes them particularly suitable for aircraft applications. Reference is made to the above statements on the first, second, third, fourth, fifth and sixth aspects of the invention, which apply analogously here.

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Examples

The present invention will now be described in detail with reference to several examples thereof. However, these examples are illustrative and do not limit the scope of the invention.

### Exemplary preparation of monomers according to the invention

The monomers according to the present invention can be prepared by means of syntheses customary in the art and familiar to the person skilled in the art. Exemplary methods of synthesis are shown below, but are not limited thereto.

### Preparation Example 1 - Monomers according to structure a)

### Example 1A

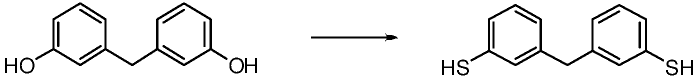

Bisphenol F (CAS 620-92-8) is commercially available. The conversion of the hydroxyl groups to the thiol groups can be conducted as described in J. Arnould et al., Tetrahedron Letters (1996), 37, 4523-4524. 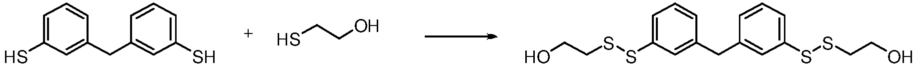 2-Mercaptoethanol (CAS 60-24-2) is commercially available. The disulfide formation can be carried out analogously to M. Pi 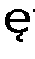 ta et al., Polym. Chem. (2023), 14, 7-31. 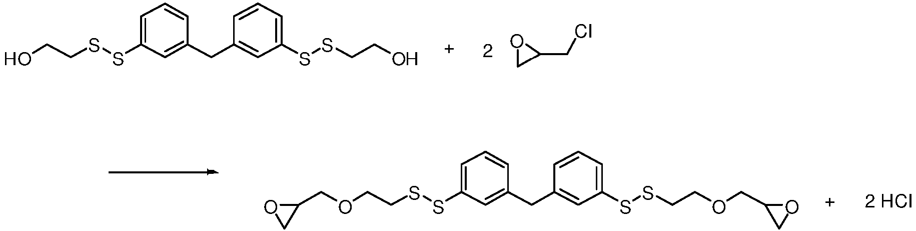 Epichlorohydrine (CAS 106-89-8) is commercially available.

### Example 1B

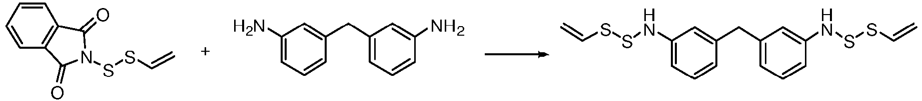

4,4'-Methylenedianiline (CAS 101-77-9) is commercially available. The other educt is available as described in H. Asanuma et al., Angew. Chem. Int. Ed. (2023), 62, e202219156. The reaction is conducted as described in H. Asanuma et al., Org. Lett. (2024), 26, 438-443.

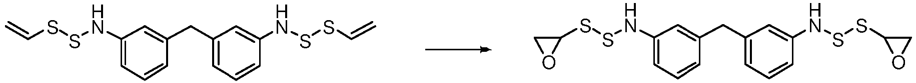

The epoxidation can be carried out using the Prileschaev reaction. 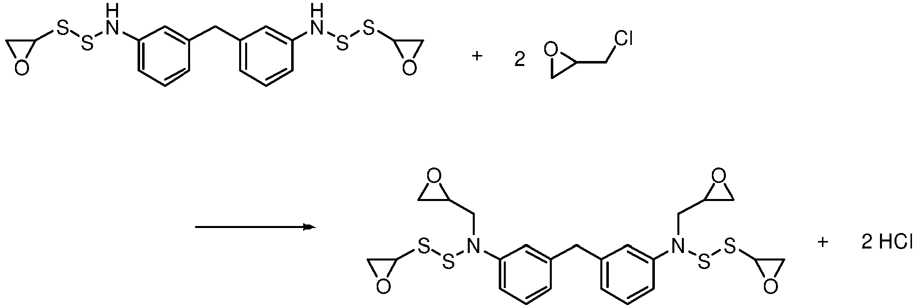 Epichlorohydrine (CAS 106-89-8) is commercially available.

### Example 1C

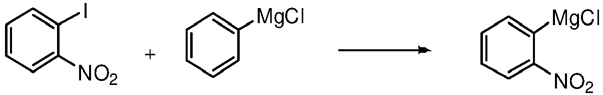

1-iodo-2-nitrobenzene (CAS 609-73-4) and Phenylmagnesium chloride (CAS 100-59-4) are commercially available. The reaction is conducted as described in I. Sapountzis et al., J. Org. Chem. (2005), 70, 2445-2454. 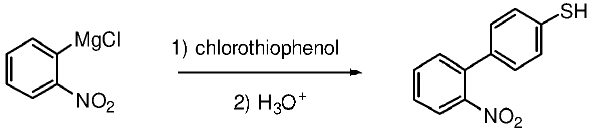 Chlorothiophenol (CAS 106-54-7) is commercially available. The reaction is conducted as described in I. Sapountzis et al., J. Org. Chem. (2005), 70, 2445-2454. 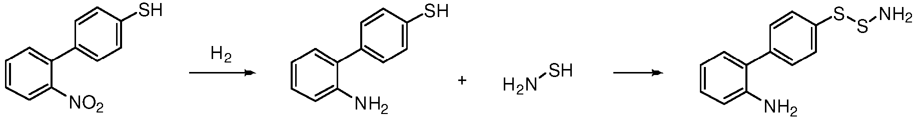 2-Aminothiophenol (CAS 137-07-5) is commercially available.

### Preparation Example 2 - Monomers according to structure b)

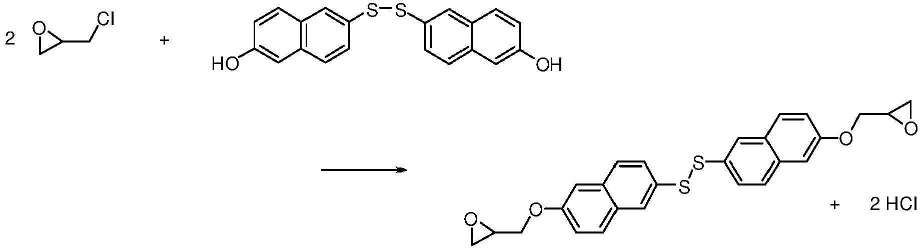

Epichlorohydrine (CAS 106-89-8) and Bis(2-hydroxy-6-naphtyl) disulfide (CAS 6088-51-3) are commercially available.

### Preparation Example 3 - Monomers according to structure c)

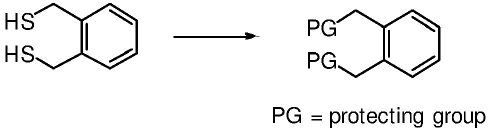

1,2-Benzoldimethanthiol (CAS 2388-68-3) is commercially available. The protection of the thiol groups is conducted as described in F. Goethals et al. Progress in Polymer Science (2017), 64, 76-113.

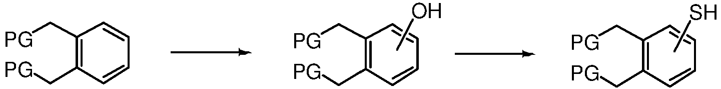

The hydroxyl group is introduced via a cumene process. The conversion of the hydroxyl group to the thiol group is conducted as described in J. Arnould et al., Tetrahedron Letters (1996), 37, 4523-4524.

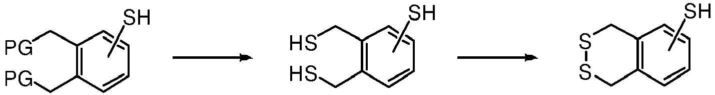

The deprotection of the thiol groups is conducted as described in F. Goethals et al. Progress in Polymer Science (2017), 64, 76-113. The cyclisation with formation of the disulfide group can be carried out analogously to M. Pi 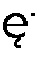 ta et al., Polym. Chem. (2023), 14, 7-31.

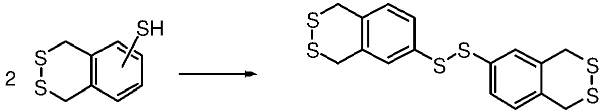

The disulfide formation can be carried out analogously to M. Pi 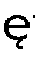 ta et al., Polym. Chem. (2023), 14, 7-31.

### Exemplary preparation of vitrimers and composites according to the invention

The vitrimers and composites according to the present invention can be prepared by means of polymerization, cross-linking and curing techniques customary in the art and familiar to the person skilled in the art. The monomers according to the present invention are polymerized and/or cross-linked and/or cured by methods as described above. For example, one or more of the monomers according to the invention are mixed, optionally with a cross-linker or hardener as described above, and the mixture is cured at temperatures between room temperature (22 °C) and 300 °C and in particular at temperatures between 80 °C and 200 °C. The prepared vitrimers and polymeric networks showed improved vitrimeric functions as well as improved strength, stiffness and Tg.

### LIST OF REFERENCE SIGNS

1 providing at least one monomer according to the first aspect of the invention
2 polymerizing, co-polymerizing and/or cross-linking the at least one monomer to obtain the polymeric network and/or vitrimer

## Claims

1. A monomer, in particular for the formation of a polymeric network and/or vitrimer, comprising or consisting of at least one of the following structures a) - c):
a) 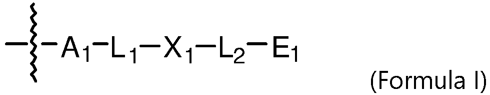 wherein
A₁ is selected from the group consisting of 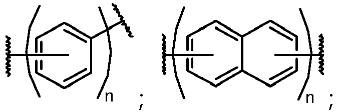 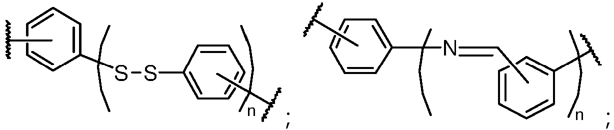 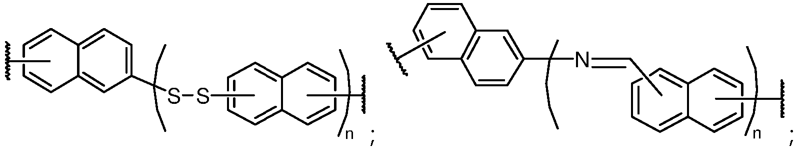 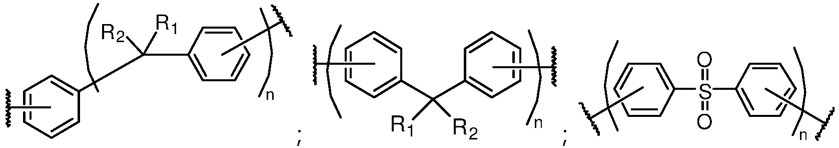 and combinations thereof, with n being an integer from 1 to 10, preferably from 1 to 3, and R₁ and R₂ are independently selected from H, C₁ to C₆ alkyl groups and C₆ to C₂₀ aryl groups;
L₁ is selected from the group consisting of a bond, 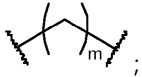 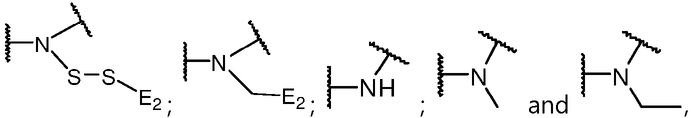
with m being an integer from 1 to 5, preferably 1 or 2, and E₂ being 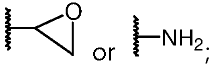
X₁ is 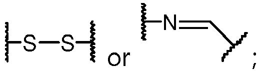
L₂ is a bond or 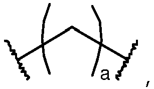 with a being an integer from 1 to 5, preferably 1 or 2; and
E₁ is 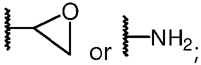 and/or
b) 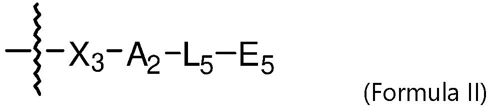 wherein
A₂ is selected from the group consisting of 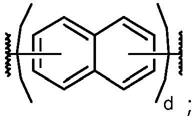 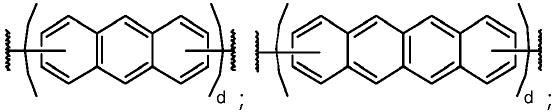 and combinations thereof, with d being an integer from 1 to 10, preferably from 1 to 3;
X₃ is 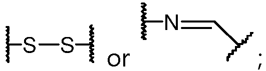
L₅ is selected from the group consisting of a bond, 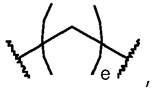 with e being an integer from 1 to 5, preferably 1 or 2; 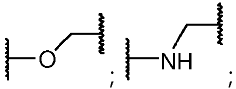 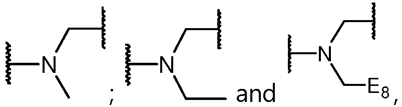 with E₈ being 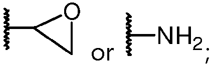 and
E₅ is 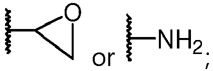 and/or
c)
G₂-Z-G₁ (Formula III)
wherein
Z is selected from the groups consisting of 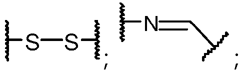 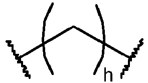 with h being an integer from 1 to 5, preferably 1 or 2; 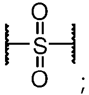 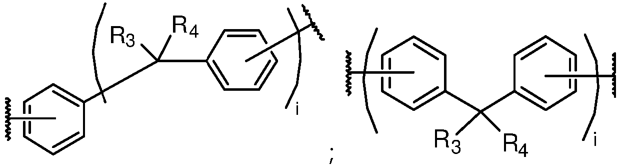 and 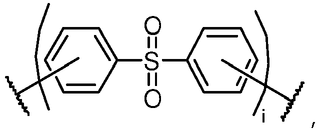 with i being an integer from 1 to 5, preferably 1 or 2, and R₃ and R₄ are independently selected from H, C₁ to C₆ alkyl groups and C₆ to C₂₀ aryl groups; and
G₁ and G₂ are each independently selected from the group consisting of 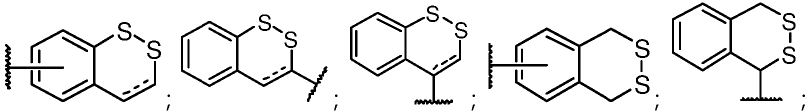 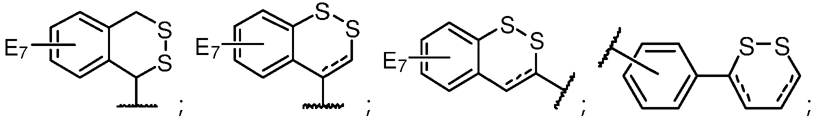 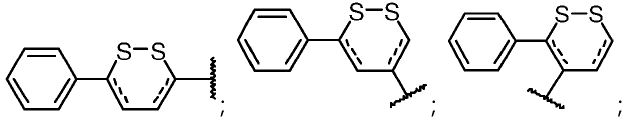 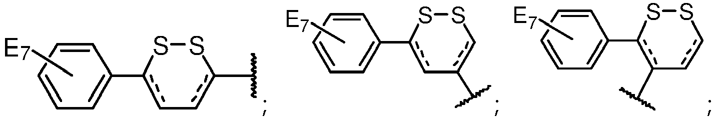 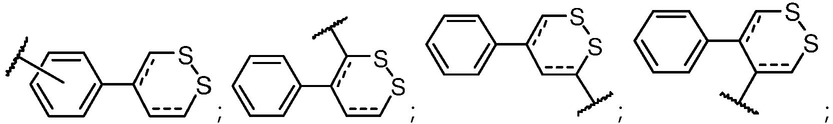 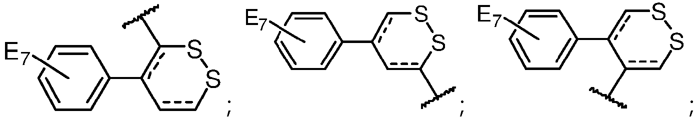 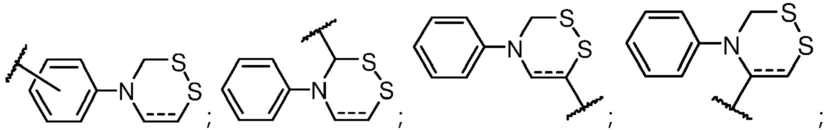 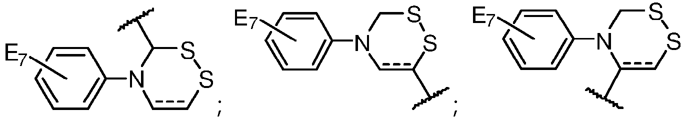 and analogue structures with four-, five-, seven- and/or eight-membered rings, preferably five- and/or seven-membered rings, wherein E₇ is selected from the group consisting of 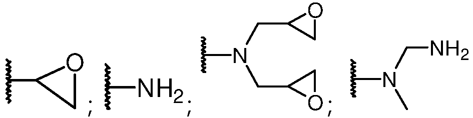 and 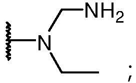
optionally wherein the aromatic and/or aliphatic groups of the structures a)-c) are optionally substituted with C₁ to C₆ alkyl groups and/or C₆ to C₂₀ aryl groups,
wherein 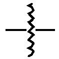 means that the structure is bound to a residue or a surface.

2. The monomer according to claim 1, wherein structure a) is a compound according to Formula (la) or Formula (Ib):
E₃-L₄-X₂-L₃-A₁-L₁-X₁-L₂-E₁ (Formula Ia),
or
E₃-A₁-L₁-X₁-L₂-E₁ (Formula Ib)
wherein A₁, L₁, X₁, L₂ and E₁ are as defined above,
L₃ is selected from the group consisting of a bond, 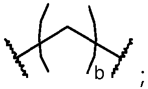 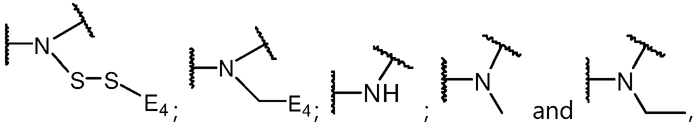 with b being an integer from 1 to 5, preferably 1 or 2, and E₄ being 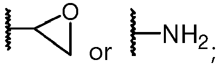
X₂ is 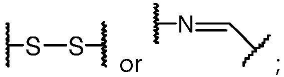
L₄ is a bond or 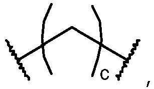 with c being an integer from 1 to 5, preferably 1 or 2; and
E₃ is selected from the group consisting of 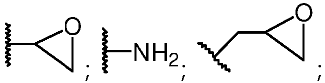 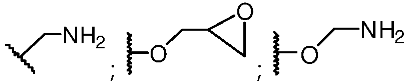 and H.

3. The monomer according to claims 1 or 2, wherein structure b) is a compound according to Formula (IIa):
E₆-L₆-A₃-X₃-A₂-L₅-E₅ (Formula (IIa)
wherein X₃, A₂, L₅ and E₅ are as defined above,
A₃ is selected from the group consisting of 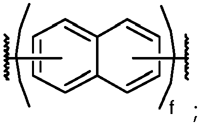 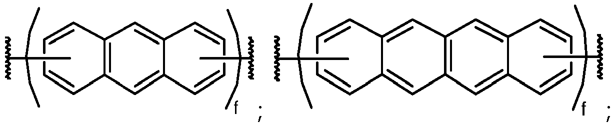 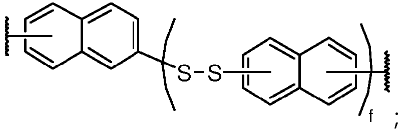 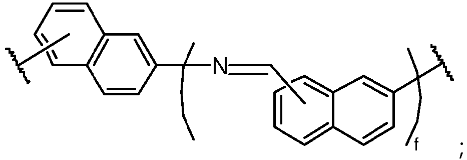 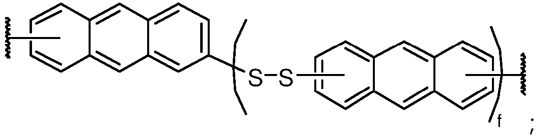 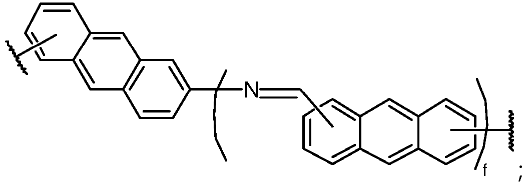 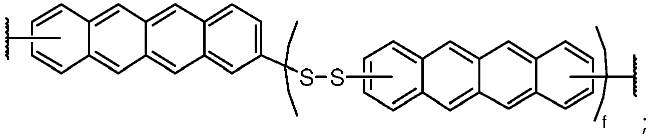 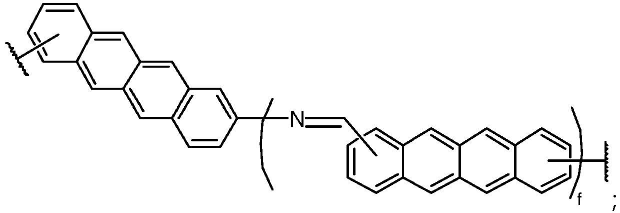 and combinations thereof, with f being an integer from 1 to 10;
L₆ is selected from the group consisting of a bond; 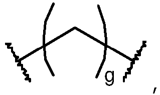 with g being an integer from 1 to 5, preferably 1 or 2; 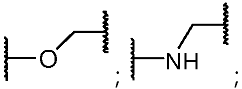 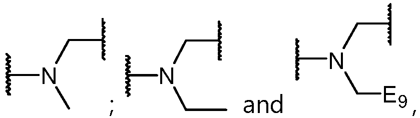 with E₉ being 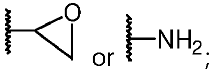 and
E₆ is 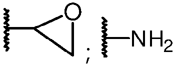 or H.

4. The monomer according to any one of the preceding claims, wherein A₁ is selected from the group consisting of 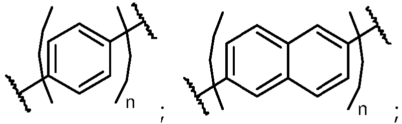 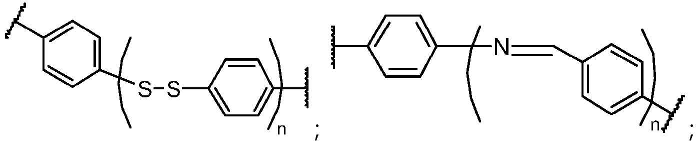 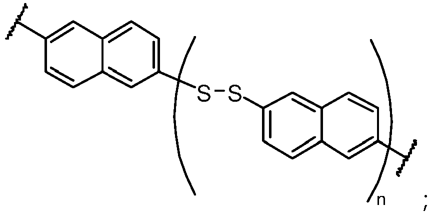 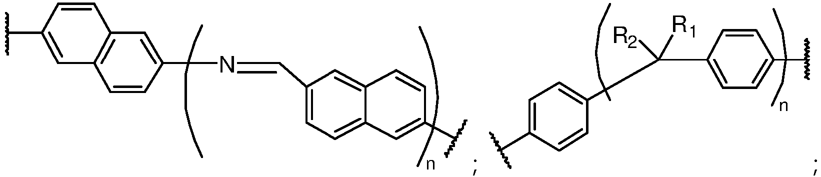 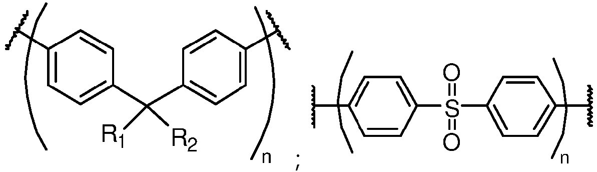 and combinations thereof, preferably wherein A₁ is selected from the group consisting of 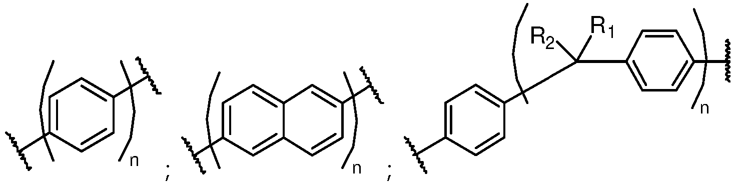 and 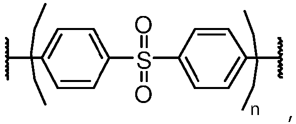 optionally wherein R₁ and R₂ are independently selected from H and C₁ to C₃ alkyl groups.

5. The monomer according to any one of the preceding claims, wherein A₂ is selected from the group consisting of 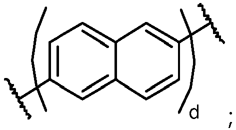 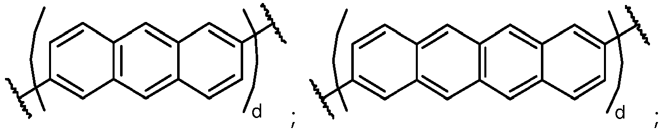 and combinations thereof, preferably wherein A₂ is selected from the group consisting of

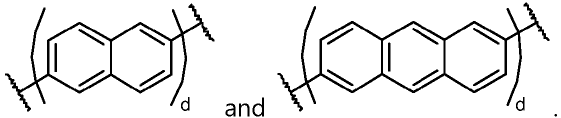

6. The monomer according to claim 3, wherein A₃ is selected from the group consisting of 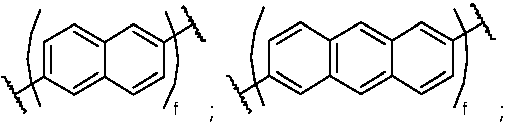 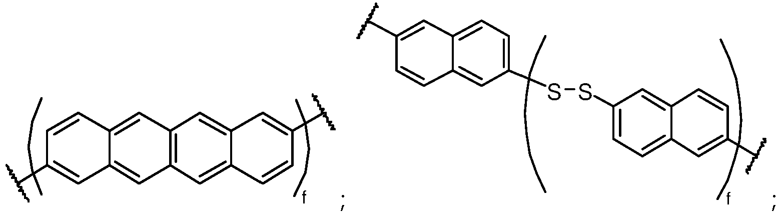 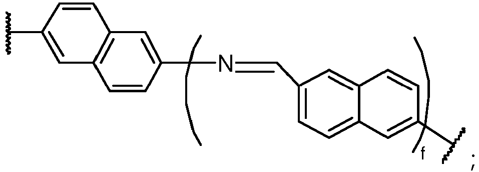 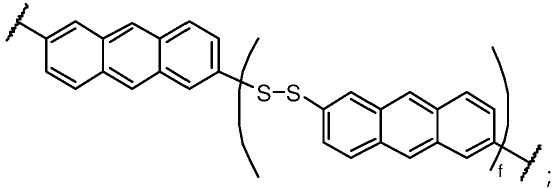 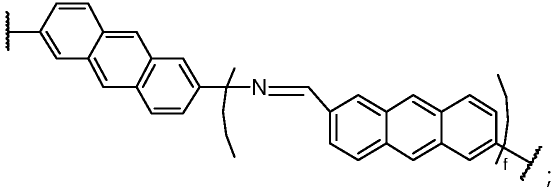 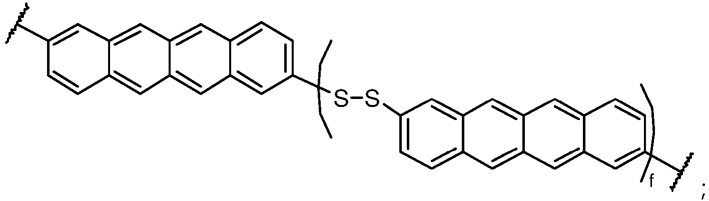 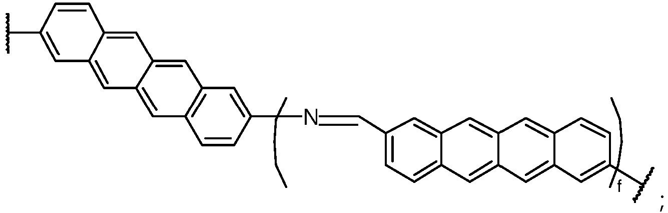 and combinations thereof, preferably wherein A₃ is selected from the group consisting of 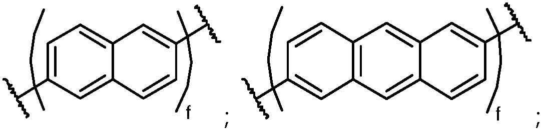 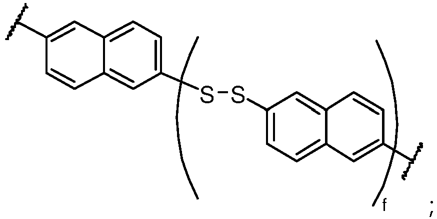 and

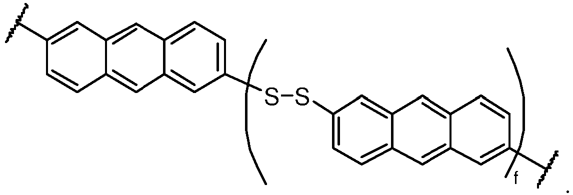

7. The monomer according to any one of the preceding claims, wherein Z is selected from the groups consisting of 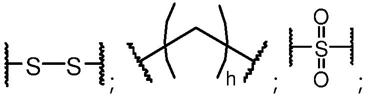 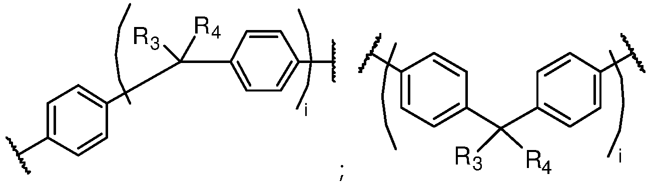 and 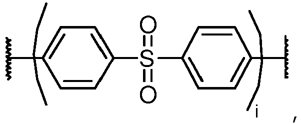 wherein R₃ and R₄ are independently selected from H and C₁ to C₃ alkyl groups, preferably wherein Z is selected from the group consisting of 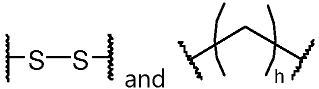 with h being 1 or 2; and/or
wherein G₁ and G₂ are each independently selected from the group consisting of 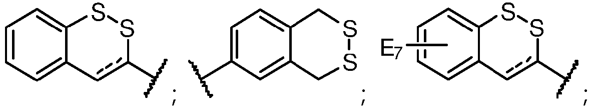 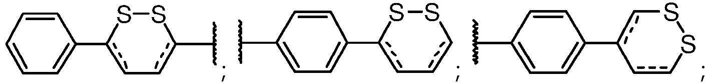 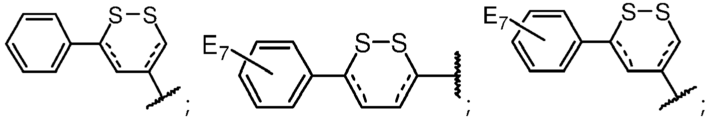 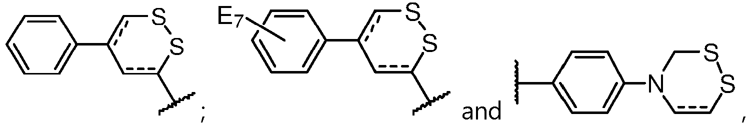 preferably wherein G₁ and G₂ are each independently selected from the group consisting of

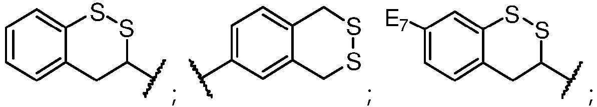

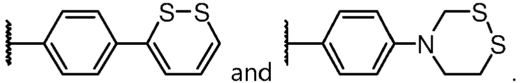

8. The monomer according to any one of the preceding claims, wherein E₁, E₂, E₄, E₅, E₆, E₈ and/or E₉ is 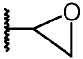 and/or wherein E₃ is selected from the group consisting of 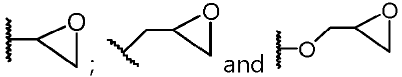 and/or wherein E₇ is

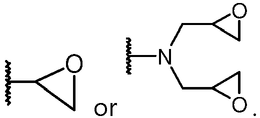

9. The monomer according to any one of the preceding claims, wherein X₁, X₂ and/or X₃ are

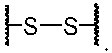

10. The monomer according to any one of the preceding claims, wherein the monomer comprises or consists of structure a) and structure a) is selected from the group consisting of the following compounds: 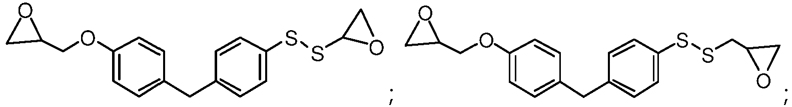 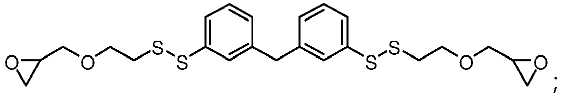 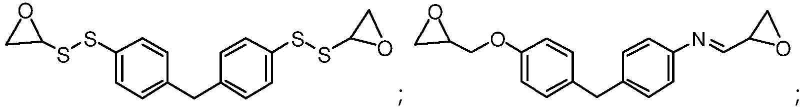 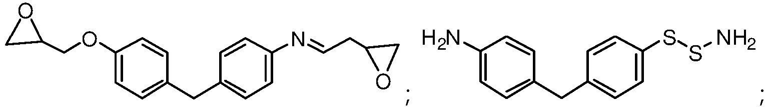 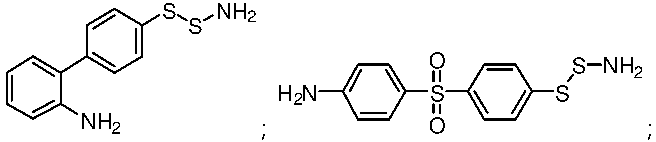 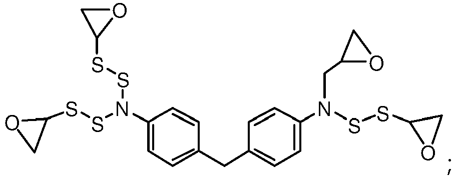 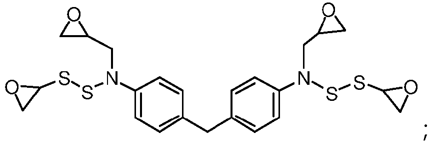 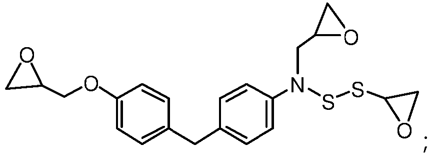 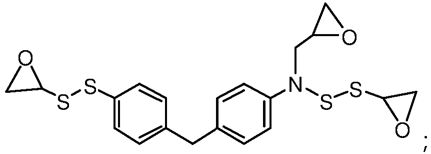 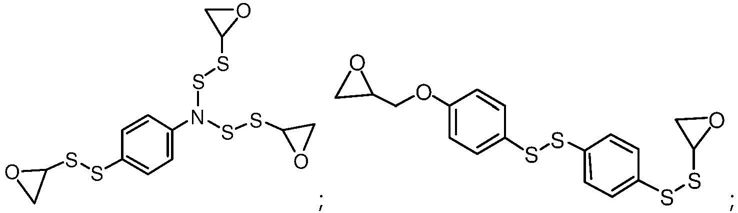 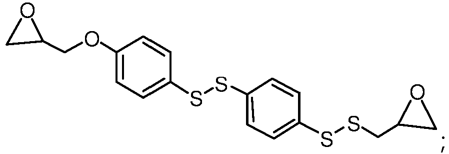 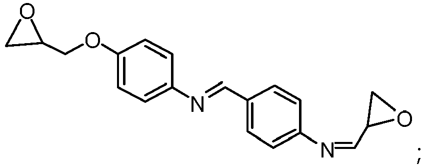 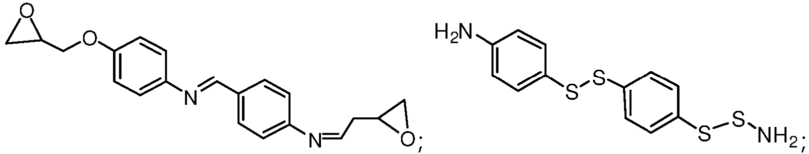 and

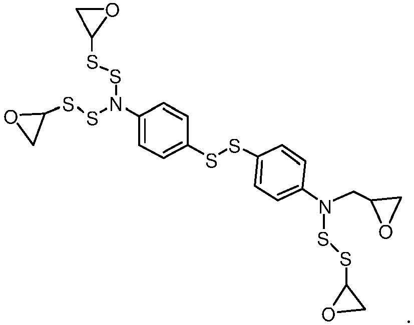

11. The monomer according to any one of the preceding claims, wherein the monomer comprises or consists of structure b) and structure b) is selected from the group consisting of the following compounds: 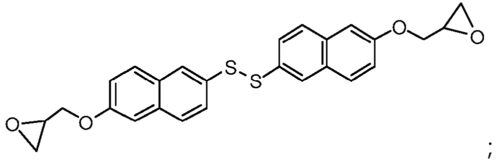 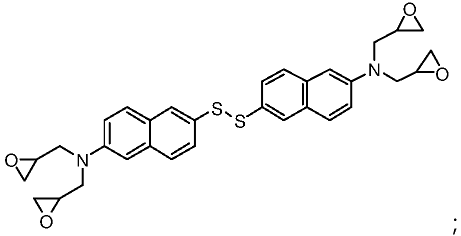 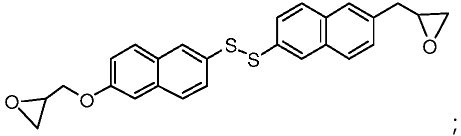 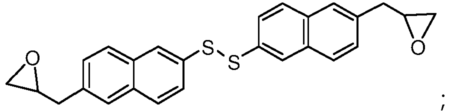 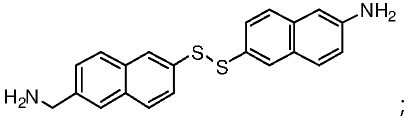 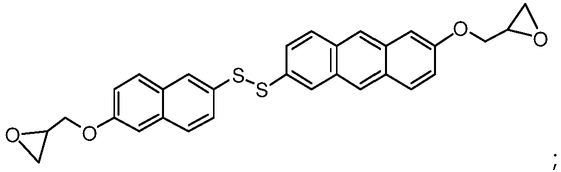 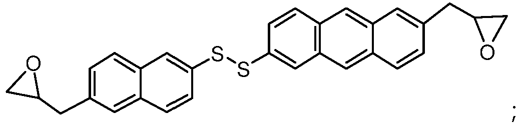 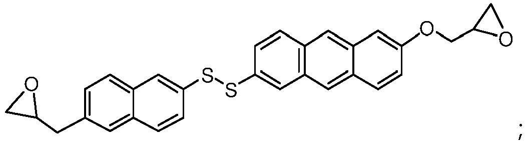 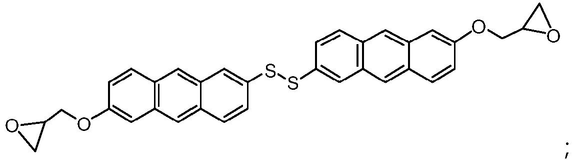 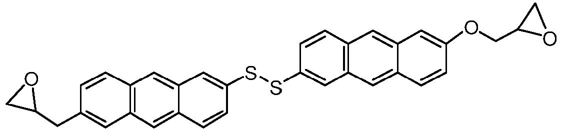 and

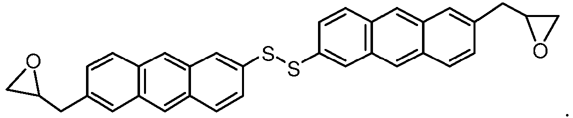

12. The monomer according to any one of the preceding claims, wherein the monomer comprises or consists of structure c) and structure c) is selected from the group consisting of the following compounds:

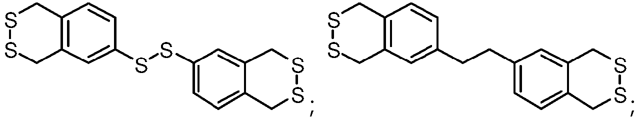

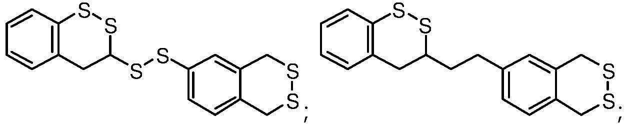

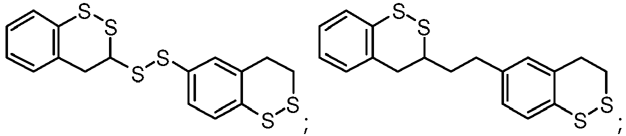

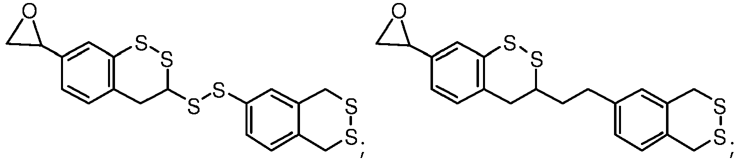

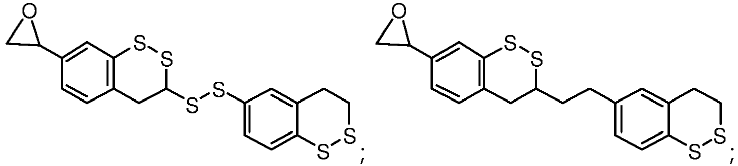

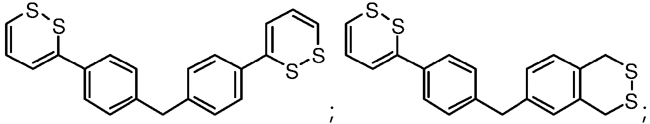

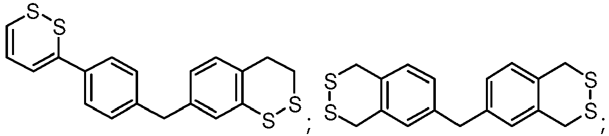

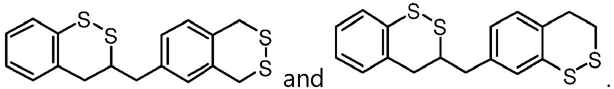

13. A method of manufacturing a polymeric network and/or vitrimer, the method comprising:
providing at least one monomer according to anyone of claims 1 to 12, polymerizing, co-polymerizing and/or cross-linking the at least one monomer to obtain the polymeric network and/or vitrimer.

14. A vitrimer or composite, produced using one or more of the monomers according to claims 1 to 12.

15. Use of a vitrimer according to claim 14 as adhesive, matrix resin or for surface functionalization, in particular in aircraft composites.
